(19) **Europäisches Patentamt**
**European Patent Office**
**Office européen des brevets**

(11) **EP 4 609 849 A1**

(12) **EUROPEAN PATENT APPLICATION**
published in accordance with Art. 153(4) EPC

(43) Date of publication:
03.09.2025 Bulletin 2025/36

(21) Application number: 23882559.0

(22) Date of filing: 20.10.2023

(51) International Patent Classification (IPC):
*A61K 8/81* (2006.01)  *A61K 8/06* (2006.01)
*A61Q 1/00* (2006.01)  *A61Q 5/00* (2006.01)
*A61Q 17/04* (2006.01)  *A61Q 19/00* (2006.01)

(52) Cooperative Patent Classification (CPC):
A61K 8/06; A61K 8/81; A61Q 1/00; A61Q 5/00;
A61Q 17/04; A61Q 19/00

(86) International application number:
PCT/JP2023/038015

(87) International publication number:
WO 2024/090354 (02.05.2024 Gazette 2024/18)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.10.2022 JP 2022172405

(71) Applicant: Toagosei Co., Ltd.
Minato-ku
Tokyo 105-8419 (JP)

(72) Inventor: SHIBATA, Hiroyuki
Nagoya-shi, Aichi 455-0026 (JP)

(74) Representative: TBK
Bavariaring 4-6
80336 München (DE)

(54) **AQUEOUS POLYMER EMULSION FOR COSMETIC, METHOD FOR PRODUCING SAME, AND COSMETIC**

(57) An aqueous polymer emulsion for cosmetics contains resin particles. The resin particles include a structural unit derived from a monomer (A) in an amount of 65 to 99.9 mass%, and a structural unit derived from a monomer (B) in an amount of 0.1 to 10 mass%, with respect to the total amount of the structural units derived from the monomers forming the resin particles as 100 mass%, wherein the monomer (A) is an alkyl (meth) acrylate having an C3 to C8 alkyl group and the monomer (B) is at least one monomer selected from the group consisting of an unsaturated carboxylic acid and an unsaturated carboxylic anhydride; and the resin particles have a volume average particle size of 70 to 150 nm and a ratio of the volume average particle size to a cumulative average particle size (volume average particle size/cumulative average particle size) of 1.00 to 1.30.

EP 4 609 849 A1

**Description**

Technical Field

[Cross-Reference to Related Applications]

The present application claims a priority from Japanese Patent Application No. 2022-172405 filed on October 27, 2022, the entirety of the disclosure of which is incorporated herein by reference.

The present disclosure relates to an aqueous polymer emulsion for a cosmetic, to a method for producing the same, and to a cosmetic.

Background Art

In cosmetics such as a makeup cosmetic composition, a manicure preparation, a skin-care cosmetic composition, and a hair cosmetic composition, a water-soluble polymer has been used as a coating film-forming agent, from environmental and hygienic aspects. However, in recent years, an aqueous polymer emulsion is widely used as a coating film-forming agent instead of a water-soluble polymer having poor moisture resistance and water resistance.

From the viewpoint of usability of cosmetics and for daily UV-protection purpose, there is demand for cosmetics exhibiting a persistent cosmetic effect. Particularly in a hot and humid summer season and while playing sports, there is demand for cosmetics that are more excellent in water resistance, oil resistance (anti-sebum performance), and persistency in cosmetic effects (hereinafter may also be referred to as "cosmetic persistency"). However, a coating film formed from a conventionally employed aqueous polymer emulsion readily causes phenomena such as braking or peeling while wet, resulting in insufficient water resistance. Also, the coating film becomes cakey or runs by sebum, sweat, an oily component of a differently applied cosmetic, or the like, resulting in coming-off of the cosmetic. Thus, oil resistance is also insufficient.

In order to overcome these drawbacks, there have been proposed countermeasures, for example, an aqueous emulsion of a resin polymerized without use of an emulsifier (see Patent Document 1), an aqueous polymer emulsion employing a silane coupling agent (see Patent Document 2), and a technique of incorporating organopolysiloxane particles (see Patent Document 3).

Prior Art Documents

Patent Documents

Patent Document 1: Japanese Patent Application Laid-Open (kokai) No. 1979-049338
Patent Document 2: Japanese Patent Application Laid-Open (kokai) No. 2002-327019
Patent Document 3: Japanese Patent Application Laid-Open (kokai) No. 2000-355532

Summary of the Invention

Problems to be Solved by the Invention

However, the aqueous polymer emulsion of Patent Document 1 exhibits insufficient stability over time, and cosmetic persistency of a cosmetic composition obtained from the polymer emulsion is unsatisfactory. Problematically, the aqueous polymer emulsion of Patent Document 2 exhibits insufficient stability over time, although cosmetic persistency of a cosmetic composition obtained from the polymer emulsion is satisfactory. In the aqueous polymer emulsion of Patent Document 3, organopolysiloxane particles and coating film-forming particles are present as a particle phase in the aqueous phase. Thus, a target coating film-forming performance cannot satisfactorily be attained, resulting in poor oil resistance in some cases.

Also, a conventional aqueous polymer emulsion has such a softness as to follow the facial expressions (i.e., motion of the skin). Thus, the polymer emulsion is sticky and provides a user with sticky sensation, and some consumers feel unpleasant sensation in use. Notably, when consumers choose or continuously buy cosmetics, one of the important key factors is sensation in use.

Further, such a conventional aqueous polymer emulsion generally contains microamounts of unreacted monomers and volatile organic compounds such as a decomposition product generated during polymerization, evoking, for example, the following problems. When the user applies the cosmetic such as a cosmetic base, a foundation, or a cream to the skin, an odor intrinsic to a volatile organic compound must be masked with a perfume or the like, in order to prevent provision of the user with the mal-odor (hereinafter may also be referred simply as "odor"). In this case, difficulty is encountered in provision of unscented cosmetics. Also, use of such conventional cosmetics is limited, when they are uses as point-make products such as cheek, mascara, and eyeshadow. Thus, there is room for further improvement of such a conventional aqueous polymer emulsion.

The present disclosure has been conceived in view of the foregoing. Thus, a main object of the disclosure is to provide an aqueous polymer emulsion for cosmetics, which emulsion can form a coating film having excellent water resistance and oil resistance, exhibits excellent low-odor performance, and can provide a cosmetic composition exhibiting excellent stability over time, softness, sensation in use, and cosmetic persistency. Another object is to provide a cosmetic composition containing the aqueous polymer emulsion.

Means for Solving the Problems

The present inventor has conducted extensive studies to solve the aforementioned problems, and has found that the problems can be solved by, in an aqueous polymer emulsion containing resin particles, regulating parameters in relation to an average particle size of the resin particles to fall within specific ranges. Accordingly, the present disclosure provides the following means.

[1] An aqueous polymer emulsion for cosmetics containing resin particles, wherein the resin particles include a structural unit derived from a monomer (A) in an amount of 65 to 99.9 mass%, and a structural unit derived from a monomer (B) in an amount of 0.1 to 10 mass%, with respect to the total amount of the structural units derived from the monomers forming the resin particles as 100 mass%, wherein

the monomer (A) is an alkyl (meth)acrylate having an C3 to C8 alkyl group; and
the monomer (B) is at least one monomer selected from the group consisting of an unsaturated carboxylic acid and an unsaturated carboxylic anhydride; and
the resin particles have a volume average particle size of 70 to 150 nm and a ratio of the volume average particle size to a cumulative average particle size (volume average particle size/cumulative average particle size) of 1.00 to 1.30.

[2] The aqueous polymer emulsion for cosmetics as described in [1] above, wherein the polymer forming the resin particles has a glass transition temperature of -20°C to 20°C.

[3] The aqueous polymer emulsion for cosmetics as described in [1] or [2] above, wherein the monomer (B) is methacrylic acid.

[4] The aqueous polymer emulsion for cosmetics as described in any of [1] to [3] above, which emulsion has a volatile organic compound level of 100 ppm or lower.

[5] The method for producing the aqueous polymer emulsion for cosmetics as recited in any of [1] to [4] above, the method including a first polymerization step of adding a monomer into a reaction container and conducting emulsion polymerization, under such conditions that a C1 to C4 lower alcohol is present at a level of 10,000 ppm or lower in the reaction container and an anionic surfactant is present in an amount of 0.15 to 0.80 parts by mass, with respect to the total amount of monomers used in production of the resin particles as 100 parts by mass, to thereby produce seed particles; and a second polymerization step of adding a monomer into a reaction container and conducting emulsion polymerization in the presence of the seed particles.

[6] The method as described in [5] above for producing an aqueous polymer emulsion for cosmetics, wherein, in the first polymerization step, the lower alcohol is present in the reaction container, and the lower alcohol is ethanol.

[7] A method for producing an odor-reduced aqueous polymer emulsion for cosmetics, the method including a step including placing the aqueous polymer emulsion for cosmetics produced through a production method as recited in [5] or [6] above in a processing container that can provide reduced pressure; and supplying pressurized steam to the processing container, to thereby remove a volatile organic compound present in the aqueous polymer emulsion for cosmetics, while the temperature of the emulsion in the processing container is controlled to 50°C to 85°C; the pressure in the processing container is controlled to 12 kPa to 57 kPa; and the internal state of the processing container is maintained under a water boiling condition.

[8] A cosmetic containing an aqueous polymer emulsion for cosmetics as recited in any of [1] to [4] above.
Advantageous Effects of the Invention

According to the present disclosure, there can be provided an aqueous polymer emulsion for cosmetics that can form a coating film with excellent water resistance and oil resistance as well as excellent low-odor performance. Also, according to the aqueous polymer emulsion for cosmetic of the present disclosure, there can be provided a cosmetic composition which can provide a cosmetic composition which exhibits excellent stability over time, softness, sensation in use, water resistance, oil resistance, and cosmetic persistency, and whose odor is sufficiently reduced.

Modes for Carrying Out the Invention

Hereinafter, the present disclosure will be described in detail. Notably, as used herein, the term "(meth)acrylic" encompasses acrylic or methacrylic or both, the term "(meth)acrylate" encompasses acrylate or methacrylate or both. Unless otherwise specified, an expression, the numerical range with an expression of "a to b" refers to a numerical range from a or greater and b or less. For example, "0 to 5 mass%" refers to a range of "0 mass% or more and 5 mass% or less".

<Aqueous polymer emulsion for cosmetics>

The aqueous polymer emulsion for cosmetics of the present disclosure is an emulsion in which resin particles are dispersed in a solvent mainly containing water. As used herein, the expression "a solvent mainly containing water" refers to a solvent containing water in an amount of 70 mass% or more (upper limit: 100 mass%). The solvent preferably contains water in an amount of 80 mass% or more, more preferably 90 mass% or more, still more preferably 95 mass% or more, yet more preferably 99 mass% or more. The optionally used solvent other than water may be an hydrophilic solvent such as acetone. Hereinafter, the term "aqueous polymer emulsion for cosmetics" or "aqueous polymer emulsion" may also be referred to simply as "emulsion".

(Monomers forming the resin particles)

The emulsion of the present disclosure contains resin particles. A monomer forming the resin particles (i.e., a structural unit of a polymer forming the resin particles) may be a monomer obtained from a typical fossil resource material such as petroleum, or a monomer obtained from a biomass resource that is environmental-friendly (e.g., in relation to the greenhouse effect) (hereinafter may also be referred to as a "biomass monomer"). The biomass monomer, which is used in order to suppress of dependency on fossil resource materials, is preferably used as a monomer forming the resin particles.

The biomass monomer is synthesized from a biomass material; i.e., a material originating from a renewable organic resource. The term biomass material typically refers to a material derived from a bio-material which can be reproduced in a sustainable manner essentially in the presence sunlight, water, and carbon dioxide (typically, a plant undergoing photosynthesis). Therefore, a material derived from a fossil resource exhausted through use after mining (i.e., a fossil resource-based material) is excluded from the concept of biomass material used in the specification. The biomass monomer content and the biomass-originating carbon content of a polymer formed through polymerization of a biomass monomer can be estimated through a $^{14}C$ isotope content as measured on the basis of ASTM D6866.

From the viewpoint of environmental consciousness, the ratio of biomass-originating carbon contained in the resin particles is preferably higher. When a biomass monomer is used as a monomer forming the resin particles, the biomass-originating carbon content is, for example, 10% or higher, preferably 20% or higher, more preferably 30% or higher, still more preferably approximately 100%.

The resin particles include, as a structural unit derived from a monomer forming the resin particles, a structural unit derived from (A) an alkyl (meth)acrylate having an C3 to C8 alkyl group (hereinafter may also be referred to as a "monomer (A)") and (B) at least one monomer selected from the group consisting of an unsaturated carboxylic acid and an unsaturated carboxylic anhydride (hereinafter may also be referred to as a "monomer (B)").

Specific examples of the monomer (A) include n-propyl (meth)acrylate, i-propyl (meth)acrylate, n-butyl (meth) acrylate, i-butyl (meth)acrylate, s-butyl (meth)acrylate, t-butyl (meth)acrylate, 2-ethylhexyl (meth)acrylate, and n-octyl (meth)acrylate. These monomers may be used singly or in combination of two or more species. Of these, the monomer (A) is preferably an alkyl (meth)acrylate having a C4 to C6 alkyl group from the viewpoint of achieving more suitable water resistance and oil resistance of the emulsion formed therefrom. From the viewpoint of achieving enhancement in water resistance and oil resistance of the emulsion at low cost, the monomer (A) preferably includes n-butyl (meth)acrylate.

Specific examples of the monomer (B) include acrylic acid, methacrylic acid, crotonic acid, maleic acid, fumaric acid, itaconic acid, citraconic acid, cinnamic acid, and maleic anhydride. These monomers may be used singly or in combination of two or more species. From the viewpoint of enhancement of water resistance and oil resistance of the emulsion at low cost, the monomer (B) preferably is preferably acrylic acid or methacrylic acid, with methacrylic acid being more preferred.

The resin particles include a structural unit derived from the monomer (A) (hereinafter may also be referred to as "monomer (A) unit") in an amount of 65 to 99.9 mass%, and a structural unit derived from the monomer (B) (hereinafter may also be referred to as "monomer (B) unit") in an amount of 0.1 to 10 mass%, with respect to the total amount of the structural units derived from the monomers forming the resin particles as 100 mass%. By controlling the monomer (A) unit content and the monomer (B) unit content of the resin particles to fall within the above ranges, water resistance, oil resistance, cosmetic persistency, and stability over time can be improved in a well-balanced manner.

More specifically, the monomer (A) unit content of the resin particles is preferably 75 mass% or more, with respect to the total amount of the structural units derived from the monomers forming the resin particles, more preferably 85 mass% or more, still more preferably 90 mass% or more, yet more preferably 93 mass% or more, from the viewpoint of achieving suitable water resistance and cosmetic persistency. The upper limit of the monomer (A) unit content is preferably 99.5 mass% or less, with respect to the total amount of the structural units derived from the monomers forming the resin particles, more preferably 99 mass% or less, still more preferably 98 mass% or less, from the viewpoint of achieving suitable oil resistance and sensation in use of the provided cosmetic composition.

The monomer (B) unit content of the resin particles is preferably 0.5 mass% or more, with respect to the total amount of the structural units derived from the monomers forming the resin particles, more preferably 1 mass% or more, still more preferably 2 mass% or more, from the viewpoint of achieving suitable stability over time, cosmetic persistency, and sensation in use of the provided cosmetic composition. The upper limit of the monomer (B) unit content is preferably 7 mass% or less, with respect to the total amount of the structural units derived from the monomers forming the resin particles, more preferably 6 mass% or less, from the viewpoint of achieving suitable water resistance and oil resistance.

The resin particles may further include a structural unit derived from a monomer differing from the monomers (A) and (B) (hereinafter may also be referred to as an "additional monomer"). Examples of the additional monomer include alkyl (meth)acrylate compounds having a C1 or C2 alkyl group such as methyl (meth)acrylate and ethyl (meth)acrylate; alkyl (meth)acrylate compounds having a C≥9 alkyl group such as lauryl (meth)acrylate, stearyl (meth)acrylate, cetyl-eicosyl (meth)acrylate, and behenyl (meth)acrylate; radical-polymerizable compounds having a cyclic structure such as styrene, vinyltoluene, divinyltoluene, α-methylstyrene, p-methylstyrene, chlorostyrene, vinyldibenzyl chloride, benzyl (meth)acrylate, cyclohexyl (meth)acrylate, and isobornyl (meth)acrylate; hydroxy group-containing vinyl compounds such as 2-hydroxyethyl (meth)acrylate, 2-hydroxypropyl (meth)acrylate, and 2-hydroxy-3-phenoxypropyl (meth)acrylate; amino group-containing vinyl compounds such as dimethylaminomethyl (meth)acrylate, diethylaminomethyl (meth)acrylate, 2-dimethylaminoethyl (meth)acrylate, 2-diethylaminoethyl (meth)acrylate, 2-(di-n-propylamino)ethyl (meth)acrylate, 2-dimethylaminopropyl (meth)acrylate, 2-diethylaminopropyl (meth)acrylate, 2-(di-n-propylamino)propyl (meth)acrylate, 3-dimethylaminopropyl (meth)acrylate, 3-diethylaminopropyl (meth)acrylate, and 3-(di-n-propylamino)propyl (meth)acrylate; amide group-containing vinyl compounds such as (meth)acrylamide, N,N-dimethyl(meth)acrylamide, and N,N-dimethylaminopropyl(meth)acrylamide; sulfonic acid group-containing vinyl compounds such as methallylsulfonic acid and vinylsulfonic acid; polyoxyalkylene group-containing vinyl compounds such as an alcohol (meth)acrylate ester having a polyoxyethylene group or a polyoxypropylene group or both; alkoxy group-containing vinyl compounds such as 2-methoxyethyl (meth)acrylate, 2-ethoxyethyl (meth)acrylate, 2-(n-propoxy)ethyl (meth)acrylate, 2-(n-butoxy)ethyl (meth)acrylate, 3-methoxypropyl (meth)acrylate, 3-ethoxypropyl (meth)acrylate, 2-(n-propoxy)propyl (meth)acrylate, and 2- (n-butoxy)propyl (meth)acrylate; cyano group-containing (meth)acrylate ester compounds such as cyanomethyl (meth)acrylate, 1-cyanoethyl (meth)acrylate, 2-cyanoethyl (meth)acrylate, 1-cyanopropyl (meth)acrylate, 2-cyanopropyl (meth)acrylate, 3-cyanopropyl (meth)acrylate (meth)acrylate, 4-cyanobutyl (meth)acrylate, 6-cyanohexyl (meth)acrylate, 2-ethyl-6-cyanohexyl (meth)acrylate, and 8-cyanooctyl (meth)acrylate; cyanated vinyl compounds such as acrylonitrile, methacrylonitrile, and ethacrylonitrile; and vinyl ether compounds such as vinyl methyl ether, vinyl ethyl ether, vinyl n-butyl ether, vinyl phenyl ether, and vinyl cyclohexyl ether. These monomers may be used singly or in combination of two or more species.

The relative amount of the structural units derived from the additional monomer in the resin particles is preferably 30 mass% or less, with respect to the total amount of the structural units derived from the monomers forming the resin particles, more preferably 20 mass% or less, still more preferably 10 mass% or less, yet more preferably 5 mass% or less, further more preferably 1 mass% or less.

(Volume average particle size and particle size distribution factor)

The resin particles contained in the emulsion of the present disclosure has a volume average particle size of 70 to 150 nm and a ratio of volume average particle size to cumulative average particle size (volume average particle size/cumulative average particle size) (hereinafter may also be referred to as a "particle size distribution factor") of 1.00 to 1.30.

The volume average particle size is an average size of particles weighted by volume and is defined by the following numerical expression (1):

$$\text{Volume average particle size} = \Sigma(V_1 \times d_1 + V_2 \times d_2 + \cdots + V_i \times d_i + \cdots + V_n \times d_n) / \Sigma(V_1 + V_2 + \cdots + V_i + \cdots + V_n) = \Sigma(V_i \times d_i)/\Sigma(V_i) \cdots (1) \tag{1}$$

wherein V represents a volume of particle, n represents the number of particles, and d represents a particle size.

Cumulative average particle size is defined through the following procedure. Firstly, a set of particles is assumed, and the total volume of the set is defined as 100%. A cumulative curve of the set is drawn. The diameter at the center of the cumulative curve is defined as a cumulative average particle size (also called "50% diameter").

When the volume average particle size of the resin particles in the emulsion is less than 70 nm, water resistance is poor, and the viscosity of the aqueous polymer emulsion excessively increases. In such a case, difficulty may be encountered in production of a high-concentration cosmetic composition in some cases. In contrast, when the volume average particle size of the resin particles in the emulsion is in excess of 150 nm, water resistance and oil resistance may be insufficient in some cases. From such viewpoints, the volume average particle size of the resin particles in the emulsion of the present disclosure is preferably 75 nm or more, more preferably 80 nm or more, still more preferably 85 nm or more, yet more preferably 90 nm or more. The upper limit of the volume average particle size of the resin particles is preferably 145 nm or less, more preferably 135 nm or less, further more preferably 125 nm or less.

From the viewpoint of achieving further enhanced water resistance and oil resistance of the emulsion, the particle size distribution factor of the resin particles is preferably 1.25 or less, more preferably 1.20 or less, still more preferably 1.15 or less, yet more preferably 1.10 or less, further more preferably 1.05 or less.

In the present specification, the volume average particle size and the cumulative average particle size are measured by means of a particle size analyzer (UPA-EX150, product of MicrotracBEL Corp.). Specifically the particle sizes are determined through the following procedure. Firstly, set zero (background measurement) is conducted at 25°C in water as a dispersion medium. Next, an aqueous polymer emulsion for cosmetics is prepared so as to have a solid concentration of 10 mass%, and the emulsion is diluted with water. A small volume of the thus-obtained liquid is added dropwise to a sample cell of the aforementioned particle size analyzer. After achievement of a uniform state of the test liquid in the sample cell, sample loading is conducted, and an indicated loading index is monitored. The loading index is regulated to fall within a range of 1.0 to 2.0, and then RUN (start of measurement) is conducted. The number of runs is set to 3, and the values of volume average particle size and those of cumulative average particle size are averaged. The measurement time is set to 60 seconds, and the refractive index of the sample particles is set to 1.59. Water is used as a dispersion medium, and the refractive index of the dispersion medium is 1.33. The range of measurement is 0.0008 $\mu$m to 6.5400 $\mu$m.

Notably, for the purpose of regulating the flowability and physical properties of the emulsion and other reasons, resin particles having a volume average particle size or a particle size distribution factor or both not satisfying the aforementioned conditions may added to the emulsion, so long as the volume average particle size and the particle size distribution factor of the emulsion to which the resin particles have been added satisfy the aforementioned conditions.

(Glass transition temperature)

The glass transition temperature (Tg) of the polymer forming the resin particles is preferably -20 to 20°C. When the glass transition temperature Tg is -20°C or higher, an emulsion that can provide appropriate adhesion can be produced. In this case, the cosmetic composition obtained from the emulsion can provide suitable sensation in use. When the glass transition temperature Tg is 20°C or lower, suitable film formability can be generally secured. From such viewpoints, the glass transition temperature Tg of the polymer forming the resin particles is more preferably -15°C or higher, still more preferably -10°C or higher. The upper limit of the glass transition temperature Tg of the polymer forming the resin particles is more preferably 15°C or lower, still more preferably 10°C or lower.

Notably, the glass transition temperature Tg of the polymer is calculated by the following numerical expression (2):

$$1/Tg = (Wa/Tga) + (Wb/Tgb) + (Wc/Tgc) + \cdots \qquad (2)$$

wherein Tg represents a glass transition temperature (unit: K) of the polymer; Tga, Tgb, Tgc, etc. represent glass transition temperatures (unit: K) of homopolymers of monomers a, b, c, etc., respectively; and Wa, Wb, Wc, etc. represent mass fractions of monomers a, b, c, etc. in the copolymers, respectively.

(Emulsion production method)

The emulsion of the present disclosure can be produced by use of a mixture containing the monomer (A) and the monomer (B) through emulsion polymerization. No particular limitation is imposed on the specific production procedure, so long as an emulsion containing resin particles having a volume average particle size and a particle size distribution factor, satisfying the aforementioned conditions. The volume average particle size and the particle size distribution factor of the resin particles can be regulated to fall within ranges of interest by modifying the type, amount, and addition method of the surfactant used, and further the shape, agitation speed, etc. of a reaction container or a stirrer or both. For example, when a surfactant is fed in an initial stage (i.e., when a surfactant is added to a reaction container before initiation of polymerization), the volume average particle size of the resin particles can be sufficiently reduced in an easy manner. Furthermore, when the initially fed surfactant concentration is adjusted to be a critical micelle concentration or higher, the particle size of the resin particles can be sufficiently reduced in an easier manner.

In one exemplary method of sufficiently reducing the particle size distribution factor of the resin particles, the amount of a surfactant added to a reaction container at a timing other than initial feeding is suppressed to such a minimum level as to secure polymerization consistency. The surfactant may be added as a mixture with a monomer or monomers, a mixture with a monomer or monomers and water, or the surfactant itself. Alternatively, the surfactant may be added to a reaction container at a timing other than initial feeding so as not to elevate the number of micelles substantially greater the number of micelles formed in the initial surfactant feeding stage. In that case, the particle size distribution factor of the resin particles can also be reduced.

In order to control the volume average particle size of the resin particles contained in the emulsion of the present disclosure and to sufficiently reduce the particle size distribution factor, the emulsion of the present disclosure is preferably produced through a method including two or more polymerization steps of adding a monomer or monomers to a reaction container and conducting emulsion polymerization. More specifically, the emulsion of the present disclosure is preferably produced by forming seed particles in first-stage polymerization reaction and, after formation of seed particles, newly adding a monomer or monomers to the reaction system and conducting polymerization reaction of a second or further stage(s).

From the viewpoint of achieving suitable control of the particle size of the resin particles, the emulsion is preferably produced through, among others, a method including the following first polymerization step and the second polymerization step.

First polymerization step: adding a monomer into a reaction container and conducting emulsion polymerization, under such conditions that a C1 to C4 lower alcohol is present at a level of 10,000 ppm or lower in the reaction container and an anionic surfactant is present in a level of 0.15 to 0.80 parts by mass, with respect to the total amount of monomers used in production of the resin particles as 100 parts by mass, to thereby produce seed particles.

Second polymerization step: adding a monomer into a reaction container and conducting emulsion polymerization in the presence of the seed particles.

·First polymerization step

In the polymerization reaction in the first polymerization step (hereinafter may also be referred to as "first-stage polymerization reaction"), emulsion polymerization is conducted under such conditions that a C1 to C4 lower alcohol (hereinafter may also be referred to simply as a "lower alcohol") is present at a level of 10,000 ppm or lower in the reaction container, to thereby form seed particles. By controlling the lower alcohol level in the reaction container to fall within the above range during polymerization reaction for forming seed particles, the particle size distribution of the seed particles becomes a sharp mono-dispersion state, and the particle size distribution factor of the resin particles in the obtained emulsion can be sufficiently reduced. Notably, in the first-stage polymerization reaction, the lower alcohol level in the reaction container is essentially 10,000 ppm or lower, and the lower alcohol level may be 0 ppm (i.e., a mode in which no lower alcohol is present in the reaction container).

Examples of the lower alcohol include methanol, ethanol, n-propanol, i-propanol, n-butanol, i-butanol, s-butanol, and t-butanol. These alcohols may be used singly or in combination of two or more species. In consideration that the emulsion of the present disclosure is used as a cosmetic composition, the lower alcohol contained in the reaction container at first-stage polymerization reaction is preferably, among others, ethanol or n-butanol or both. Also, from the viewpoint of suppression of odor and a drop in stability over time of the emulsion, a lower alcohol is preferably removed, when the lower alcohol has been added to the reaction container. In this regard, ethanol is particularly preferably a lower alcohol employed in the first-stage polymerization reaction, since ethanol can be easily removed through the below-mentioned deodorization treatment.

In the first-stage polymerization reaction, from the viewpoint of achieving a sufficiently reduced particle size distribution factor of the resin particles in the emulsion, the lower alcohol level in the reaction container is preferably 9,950 ppm or lower, more preferably 7,000 ppm or lower, still more preferably 5,000 ppm or lower, yet more preferably 3,000 ppm or lower, further more preferably 2,000 ppm or lower, yet further more preferably 1,000 ppm or lower. In the first-stage polymerization reaction, emulsion polymerization is preferably conducted under the condition that a lower alcohol is present in the reaction container (i.e., in the presence of a lower alcohol). More specifically, the lower alcohol level is preferably 0.1 ppm or higher, more preferably 1.0 ppm or higher, still more preferably 5.0 ppm or higher, yet more preferably 10 ppm or higher, further more preferably 25 ppm or higher. By conducting emulsion polymerization in the presence of a lower alcohol, to thereby form seed particles, the particle size distribution factor of the resin particles contained in the emulsion of the present disclosure can be sufficiently reduced. Particularly, the effect of improving water resistance can be enhanced.

While not wishing to be bound by the present disclosure regarding the mechanism of emulsion polymerization in the presence of a lower alcohol, it is assumed that the presence of a lower alcohol leads to enhancement in solubility of a monomer(s) in solvent, to thereby slightly reduce the number of seed particles formed, whereby the amount of surfactant adsorbed on unit particle increases, to thereby stabilize the seed particles, resulting in a particle size distribution of the seed particles in a sharp mono-dispersion state. In contrast, when the lower alcohol level in the system is in excess of 10,000 ppm, conceivably, stability of the seed particles decreases in terms of an electrical charge, coagulation tends to readily occur during growth of particles.

No particular limitation is imposed on the method of adding the lower alcohol during the first-stage polymerization reaction. From the viewpoint of achieving a sufficiently reduced particle size distribution factor of the resin particles contained in the emulsion, preferably, at least a part of lower alcohol used in the first-stage polymerization reaction is added to the reaction container before initiation of polymerization and, thereafter, polymerization reaction is conducted. In this case, the lower alcohol may be added simultaneously with addition of a monomer or monomers, or separately with addition of the monomer(s). Also, in the case where initiation of polymerization under the condition in which the lower alcohol has been added in an initial stage in the first-stage polymerization reaction, a lower alcohol may be boosted thereafter. Boosting of the lower alcohol may be carried out once or more times, or through continuous dropwise addition. From the viewpoint of regulating the particle size distribution factor of the resin particles to fall within a range of interest, the entirety of the lower alcohol is preferably added to a reaction container before initiation of polymerization.

In the first-stage polymerization reaction, a lower alcohol is present in the reaction system, and at least an anionic surfactant serving as a surfactant is used in a controlled amount. More specifically, in the first-stage polymerization reaction, emulsion polymerization is conducted under such conditions that an anionic surfactant is present in a level of 0.15 to 0.80 parts by mass, with respect to the total amount of the monomer used in production of the resin particles (i.e., monomers forming the resin particles) as 100 parts by mass, to thereby produce seed particles. By use of the anionic surfactant in an amount falling within the aforementioned range, the volume average particle size of the resin particles contained in the emulsion can be regulated to an appropriate value, to thereby yield an emulsion having excellent water resistance and oil resistance. As used herein, the term "anionic surfactant" refers to a surfactant that can form ions in aqueous solution, with an anion serving as a hydrophilic moiety.

In the first-stage polymerization reaction, when the anionic surfactant level in the system is lower than 0.15 parts by mass, with respect to the total amount of the monomer used in production of the resin particles as 100 parts by mass, generally, the volume average particle size of the resin particles contained in the emulsion excessively increases, resulting in poor water resistance and oil resistance of the emulsion. Also, when the anionic surfactant level in the system is in excess of 0.80 parts by mass, with respect to the total amount of the monomer used in production of the resin particles as 100 parts by mass, generally, the volume average particle size of the resin particles contained in the emulsion excessively decreases, resulting in insufficient water resistance of the emulsion. From these viewpoints, in the first-stage polymerization reaction, the anionic surfactant level in the reaction container, with respect to the total amount of the monomer used in production of the resin particles as 100 parts by mass, is preferably regulated to 0.18 parts by mass or higher, more preferably 0.20 parts by mass or higher, still more preferably 0.25 parts by mass or higher, yet more preferably 0.30 parts by mass or higher. The upper limit of the anionic surfactant level in the reaction container, with respect to the total amount of the monomer used in production of the resin particles as 100 parts by mass, is preferably regulated to 0.78 parts by mass or lower, more preferably 0.75 parts by mass or lower.

The surfactant employed in the first-stage polymerization reaction may be an anionic surfactant as a single component, or may further include a surfactant differing from an anionic surfactant (hereinafter may also be referred to as an "additional surfactant") in combination. Examples of the additional surfactant include a nonionic surfactant, a cationic surfactant, and a polymerizable surfactant. Of these, a nonionic surfactant is preferred. As used herein, the term "nonionic surfactant" refers to a surfactant which exhibits surface activity without dissociating to ions in aqueous solution. The term "cationic surfactant" refers to a surfactant that can form ions in aqueous solution, with a cation serving as a hydrophilic moiety. The term "polymerizable surfactant" refers to an anionic or nonionic surfactant having, in the molecule thereof, one or more unsaturated double bonds which can undergo radical polymerization.

From the viewpoints of regulating the volume average particle size and the particle size distribution factor of the resin particles to fall within a specific range, to thereby yield an emulsion exhibiting properties (e.g., water resistance, heat resistance, and stability over time) improved in a well-balanced manner, the surfactant employed in the first-stage polymerization reaction preferably has an anionic surfactant content, with respect to the total amount (100 mass%) of the surfactants used in the first-stage polymerization step, of 50 mass% or more, more preferably 60 mass% or more, still more preferably 80 mass% or more, yet more preferably 90 mass% or more, further more preferably 95 mass% or more, yet further more preferably 99 mass% or more.

Specific examples of the anionic surfactant serving as the surfactant employed in emulsion polymerization for producing the emulsion of the present disclosure include alkylarylsulfonate salts such as laurylbenzenesulfonate; polyoxyethylene alkyl ether sulfate salts such as polyoxyethylene lauryl ether sulfate; alkylsulfate salts such as laurylsulfate salts; polyoxyethylene polyoxypropylene alkyl ether sulfate salts; polyoxyethylene alkyl ether acetate salts such as polyoxyethylene lauryl ether acetate salt; alkylsulfosuccinate salts such as dialkylsulfosuccinate salt, polyoxyalkylene alkylsulfosuccinate salt, and dioctylsulfosuccinate salt; polyoxyethylene styrenated phenyl ether sulfate salts; polyoxyethylene distyrenated phenyl ether sulfate salts; higher fatty acid salts such as sodium oleate; polyoxyethylene fatty acid amide ether sulfate salts; coconut acid methyltaurate salts; N-acyl-L-asparetate salts; coconut acid ethyl ester sulfonate salts; alkyl-β-alanine salts; acylmethyltaurate salts; alkylethanesulfonate salts; polyoxyethylene alkyl ether carboxylate salts; alkanesulfonate salts; olefinsulfonate salts; alkylphosphate salts; polyoxyalkylene alkyl ether phosphate salts; alkyl(or alkenyl)phosphate salts; and alkylamide phosphate salts.

Examples of the nonionic surfactant include polyoxyethylene alkyl ethers such as polyoxyethylene lauryl ether, polyoxyethylene cetyl ether, polyoxyethylene stearyl ether, polyoxyethylene oleyl ether, and polyoxyethylene behenyl ether; polyoxyethylene alkylphenyl ethers such as polyoxyethylene octylphenyl ether and polyoxyethylene nonylphenyl ether; polyoxyethylene styrenated phenyl ether; polyoxyethylene distyrenated phenyl ether; sorbitan higher fatty acid esters such as sorbitan monolaurate, sorbitan monostearate, and sorbitan trioleate; polyoxyethylene sorbitan higher fatty acid esters such as polyoxyethylene sorbitan monolaurate and polyoxyethylene sorbitan monostearate; polyoxyethylene higher fatty acid esters such as polyoxyethylene monolaurate and polyoxyethylene monostearate; glycerin higher fatty acid esters such as monoglyceride oleate and monoglyceride stearate; polyoxyethylene polyoxypropylene alkyl ethers such as polyoxyethylene-polyoxypropylene block copolymer, polyoxyethylene-polyoxypropylene cetyl ether, and polyoxyethylene-polyoxypropylene decyltetradecyl ether; diethanolamides such as coconut acid diethanolamide, laurylic acid diethanolamide, myristic acid diethanolamide, palmitic acid diethanolamide, stearic acid diethanolamide, isostearic acid diethanolamide, and oleic acid diethanolamide; monoethanolamides such as coconut acid monoethanolamide, laurylic acid monoethanolamide, myristic acid monoethanolamide, palmitic acid monoethanolamide, stearic acid monoethanolamide, isostearic acid monoethanolamide, and oleic acid monoethanolamide; isopropanolamides such as coconut acid isopropanolamide, lauric acid isopropanolamide, myristic acid isopropanolamide, palmitic acid isopropanolamide, straric acid isopropanolamide, isostearic acid isopropanolamide, and oleic acid isopropanolamide; polyoxyethylene monoethanolamides such as polyoxyethylene coconut acid monoethanolamide, polyoxyethylene laurylic acid monoethanolamide, polyoxyethylene myristic acid monoethanolamide, polyoxyethylene palmitic acid monoethanolamide, polyoxyethylene stearic acid monoethanolamide, polyoxyethylene isostearic acid monoethanolamide, and polyoxyethylene oleic acid monoethanolamide; alkyl glucosides such as decyl glucoside and lauryl glucoside; alkyldimethylamine oxides such as lauryldimethylamine oxide and stearyldimethylamine oxide; maltitol hydroxyfatty acid alkyl ethers; alkylated polysacchrides; sucrose fatty acid esters; and fatty acid isopropanolamides.

Examples of cationic surfactant include alkyl tirmethylammonium salts such as lauryl trimethylammonium chloride; dialkyldimethylammonium salts such as distearyldimethylammonium chloride; alkyldimethylethylammonium salts such as octyldimethylethylammonium chloride; trialkylbenzylammonium salts such as tributylbenzylammonium chloride; alkyldimethylbenzylammonium salts such as lauryldimethylbenzylammonium chloride; alkylamine salts such as stearylamine hydrochloride; alkyldimethylaminopropylamides such as stearyldimethylaminopropylamide; and alkylpyridinium salts such as laurylpyridinium chloride.

Examples of the polymerizable surfactant include propenylalkylsulfosuccinate ester salts, (meth)acrylic acid polyoxyethylene sulfate salts, (meth)acrylic acid polyoxyethylene phospahe salts (e.g., Eleminol RS-30, product of Sanyo Chemical Industries, Ltd.), polyoxyethylene alkylpropenylphenyl ether sulfate salts (e.g., Aqualon HS-10, product of DKS Co., Ltd.), allyloxymethylalkyloxypolyoxyethylene sulfate salts (e.g., Aqualon KH-10, product of DKS Co., Ltd.), allyloxymethylnonylphenoxyethylhydroxy polyoxyethylene sulfate salts (e.g., Adeka Reasoap SE-10, product of ADEKA Corp.), allyloxymethylalkoxyethylhydroxy polyoxyethylene sulfate salts (e.g., Adeka Reasoap SR-10 and SR-30), polyoxyalkylenealkenyl ether sulfate salts (e.g., Latemul PD-104 and PD-105, product of Kao Corporation), bis(polyoxyethylene polycyclic phenyl ether) methacrylated sulfate salts (e.g., Antox MS-60, product of Nippon Nyukazai Co., Ltd.), allyloxymethylalkoxyethylhydroxy polyoxyethylene (e.g., Adeka Reasoap ER-20, product of ADEKA Corp.), polyoxyethylene alkylpropenylphenyl ether (e.g., Aqualon RN-20, product of DKS Co., Ltd.), and allyloxymethylnonylphenoxyethylhydroxy polyoxyethylene (e.g., Adeka Reasoap NE-10, product of ADEKA Corp.).

In the first-stage polymerization reaction, no particular limitation is imposed on the method of adding the surfactant. However, from the viewpoint of achieving a sufficiently small volume average particle size of the resin particles, preferably, at least a part of the surfactant employed in the first-stage polymerization reaction is added to the reaction container before initiation of polymerization, and then polymerization reaction is performed. In that case, the surfactant may be added simultaneously addition of a monomer or monomers or at a timing different from addition of the monomer(s). Also, in accordance with need, the surfactant may be dissolved in water or the like, and the product is added thereto. In the first-stage polymerization reaction, after addition of the surfactant in an initial stage so as to initiate polymerization, boosting of surfactant may be conducted. Boosting of the surfactant may be carried out once or more times, or through continuous dropwise addition.

The relative amount of the monomer forming the seed particles, with respect to the total amount of the monomers forming the resin particles contained in the emulsion of the present disclosure, is preferably 0.1 to 30 mass%, with respect to the total amount (100 mass%) of the monomers forming the resin particles, more preferably 0.1 to 20 mass%. By regulating the amount of the monomers forming the seed particles to fall within the aforementioned range, generally, the particle size distribution factor of the obtained emulsion can be reduced. Notably, the proportion of the monomers used in production of the resin particles is substantially equivalent to those of the monomers forming the resin particles. Thus, in polymerization, feed proportions of the monomers and the ratio of the monomers used in production of the seed particles, with respect to the total amount of the monomers used in production of the resin are appropriately tuned, in accordance with target monomer proportions of the resin particles.

Formation of seed particles can be easily detected through visual observation of the change of the contents in the reaction container. Alternatively, formation of seed particles may be confirmed by reactivity of a monomer composition used in polymerization for forming the seed particles.

·Second polymerization step

The emulsion of the present disclosure is preferably produced by forming seed particles through the first-stage polymerization reaction, and then conducting second-stage or further stage of polymerization reaction. No particular limitation is imposed on the number of polymerization steps for yielding the resin particles contained in the emulsion of the present disclosure. That is, the emulsion of the present disclosure may be produced through a method including only the first and second polymerization steps employed in the polymerization step for producing the resin particles. Alternatively, the emulsion may be produced through a similar method further including a third or fourth polymerization step or the like, for the purpose of regulating the volume average particle size and the cumulative average particle size of the resin particles and the compositional proportions of the monomers or for other reasons.

No particular limitation is imposed on the timing of initiation of the second-stage polymerization reaction. From the viewpoint of yielding resin particles having an appropriate volume average particle size and a sufficiently small particle size distribution factor, the second-stage polymerization reaction is preferably initiated at a timing where the reactivity of the monomers added to the first-stage polymerization reaction has reached 50 to 100%.

The reactivity (percent reactivity) is determined through the following procedure. A sample (about 0.5 g) is taken into a weighing bottle whose weight has been measured in advance [weight of weighing bottle = B (unit: g)], and the sample and the weighing bottle are weighed together correctly [weight of sample and weighing bottle before drying = WO (unit: g)]. The weighing bottle containing the sample is placed in a non-blow drier and dried at 105°C for 5 hours. After drying, the sample and the weighing bottle are weighed together [weight of sample and weighing bottle after drying = W1 (unit: g)], and the mass of the non-volatile component of the sample [X (unit: g)] is calculated by the following numerical expression (3):

$$X \ = \ (W1-B)/(W0-B) \quad \cdots \quad (3).$$

When the theoretical mass of the non-volatile component under the assumption that all the monomer components contained in the sample have been polymerized is represented by [Y (unit: g)], reactivity is calculated by the following numerical expression (4):

$$Reactivity \ (\%) \ = \ (X/Y) \times 100 \quad \cdots \quad (4).$$

The monomer composition of the monomer(s) employed in the polymerization reaction in the second or further stage may be identical to or different from the monomer composition of the monomer(s) forming seed particles in the first-stage polymerization reaction. From the viewpoint of suitably controlling the particle size of the resin particles, preferably, one monomer is used in the first-stage polymerization reaction for forming the seed particles, and two or more monomers are used in the polymerization reaction in the second or further stage, in accordance with uses and the like of the emulsion.

No particular limitation is imposed on the method of adding a monomer or monomers in the polymerization reaction in the second or further stage, and the monomer or monomers are preferably added dropwise continuously to the reaction container in conducting polymerization reaction. In dropwise addition of the monomer or monomer(s), addition is conducted generally over 0.5 to 8 hours, preferably 1 to 6 hours. The polymerization initiator may be added simultaneously with addition of the monomer or monomer(s) or at a timing different from addition thereof. Also, in accordance with need, the polymerization initiator may be dissolved in water or the like, and the product is added thereto. Boosting of the polymerization initiator may be carried out once or more times, or through continuous dropwise addition.

The polymerization reaction in the second or further stage is preferably conducted in the presence of at least any of a surfactant and a protective colloid, more preferably in the presence of a surfactant. The surfactant employed in the polymerization reaction in the second or further stage is preferably an anionic surfactant or a nonionic surfactant or both. No particular limitation is imposed on the amount of the surfactant used in the polymerization reaction in the second or further stage, and the amount is preferably 0.1 to 20 parts by mass, with respect to 100 parts by mass of a monomer or monomers used for forming resin particles. When an appropriate amount of surfactant(s) is used, mechanical stability of the resin particles is more enhanced. When an appropriate amount of polymerizable surfactant(s) is used, mechanical stability of the resin particles is further enhanced. When the amount of the surfactant used is 0.1 parts by mass or more, suitable emulsion stability can be secured. Also, when the amount of the surfactant used is 20 parts by mass or less, suitable water resistance can be secured. Both cases are preferred.

·Emulsion polymerization

No particular limitation is imposed on the emulsion polymerization method employed in the first and second polymerization steps, so long as the aforementioned conditions are satisfied, and a known method may be employed. In the emulsion polymerization, a radical polymerization initiator (hereinafter may also be referred to simply as a "polymerization initiator") is preferably used. As the polymerization initiator, a known oil-soluble polymerization initiator or a known water-soluble polymerization initiator may be used.

Examples of the oil-soluble polymerization initiator include organic peroxides such as benzoyl peroxide, tert-butyl oxybenzoate, tert-butyl hydroperoxide, tert-butyl peroxy-2-ethylhexanoate, tert-butyl peroxy-3,5,5-trimethylhexanoate, di(tert-butyl) peroxide, cumene hydroperoxide, and p-menthane hydroperoxide; and azobis compounds such as 2,2'-azobisisobutyronitrile, 2,2'-azobis-2,4-dimethylvaleronitrile, 2,2'-azobis(4-methoxy-2,4-dimethylvaleronitrile), and 1,1'-azobiscyclohexane-1-carbonitrile.

Examples of the water-soluble polymerization initiator include ammonium persulfate, sodium persulfate, potassium persulfate, hydrogen peroxide, and 2,2'-azobis(2-methylpropionamidine) dihydrochloride.

In emulsion polymerization, a reducing agent may be used in combination with the polymerization initiator. By use of a reducing agent in combination, polymerization reaction can be accelerated. Examples of such a reducing agent include reducing organic compounds such as ascorbic acid, erythorbic acid, tartaric acid, citric acid, glucose, and a metal salt of formaldehyde bissulfoxylate or the like; reducing inorganic compounds such as sodium sulfite, sodium bisulfite, sodium metabisulfite (SMBS), and sodium hyposulfite; ferrous chloride, Rongalite, and thiourea dioxide.

In emulsion polymerization, a water-soluble polymerization initiator is preferably used as the polymerization initiator. The polymerization initiator is preferably used in an amount of 0.05 to 5 parts by mass, with respect to 100 parts by mass of a monomer or monomers used for forming resin particles. The reducing agent is preferably used in an amount of 0.01 to 2.5 parts by mass, with respect to 100 parts by mass of a monomer or monomers used for forming resin particles.

In emulsion polymerization for yielding the emulsion of the present disclosure, a buffer, a chain-transfer agent, a basic compound, or the like may be used in accordance with needs. Examples of the buffer include sodium acetate, sodium citrate, and sodium bicarbonate. Examples of the chain-transfer agent include 2-mercaptoethanol, octyl mercaptan, tert-dodecyl mercaptan, lauryl mercaptan, stearyl mercaptan, 2-ethylhexyl mercaptoacetate, octyl mercaptoacetate, 2-ethylhexyl mercaptopropionate, and octyl mercaptopropionate.

The basic compound is used for neutralization. Examples thereof include ammonia; alkylamines such as trimethylamine, triethylamine, and butylamine; alcoholamines such as 2-dimethylaminoethanol, diethylaminoethanol, diethanolamine, triethanolamine, and aminomethylpropanol; ether amines such as morpholine; basic amino acids such as arginine; and aqueous metal hydroxides such as potassium hydroxide and sodium hydroxide.

(Deodorization step)

Generally, in the emulsion produced through emulsion polymerization, a microamount of volatile organic compounds, including unpolymerized monomers and decomposition products generated in polymerization, are present. A high level of volatile organic compounds in the emulsion may undesirably cause mal-odor. Thus, preferably, the emulsion produced through emulsion polymerization is subjected to a treatment of removing volatile organic compounds present in the emulsion (also called a "deodorization treatment"), to thereby reduce the odor level.

From the viewpoint of sufficiently reducing mal-odor, the volatile organic compound level of the emulsion of the present disclosure is preferably 100 ppm or lower, more preferably 50 ppm or lower, still more preferably 20 ppm or lower, yet more preferably 10 ppm or lower. The level is preferably a value approximately 0 ppm.

Examples of the technique of reducing the volatile organic compound level include a technique which includes heating of the emulsion to thereby volatilize volatile organic compounds remaining in the emulsion; a technique which includes causing gas (e.g., air) to pass through the gas phase on the emulsion; a technique which includes blowing steam into the emulsion; and a technique which includes distillating out volatile organic compounds under reduced pressure. From the viewpoint of sufficiently lowering the volatile organic compound level in the emulsion to thereby reduce mal-odor, preferably, pressurized steam is blown under reduced pressure to the emulsion produced through the aforementioned polymerization step, to thereby reduce the volatile organic compound level in the emulsion to an extremely low level of 100 ppm or lower.

More specifically, in a preferred procedure, the emulsion produced through the aforementioned polymerization step is fed to a processing container that can provide reduced pressure. The temperature and the pressure of the emulsion in the processing container are adjusted to 50°C to 85°C and 12 kPa to 57 kPa, respectively. While the state in the processing container is maintained in a water boiling state, pressurized steam is supplied into the processing container. Also, steam and volatile organic compounds vaporized from the emulsion which stay in the gas phase of the processing container are removed to the outside of the system by means of a vacuum pump, to thereby remove the volatile organic compounds. As a result, an odor-reduced emulsion is yielded.

When the temperature of the emulsion to which steam is blown is adjusted to 50°C or higher, retardation in speed of removing the volatile organic compounds can be suppressed, whereby an odor-reduced emulsion can be efficiently produced. Also, when the temperature of the emulsion to which steam is blown is adjusted to 85°C or lower, formation of a large mass of polymer coating film on the inner wall of the processing container (in particular, at the gas-liquid phase interface) can be suppressed, whereby a drop in quality due to a decrease in non-volatile component concentration can be suppressed.

In order to maintain the water boiling state, the pressure in the processing container is preferably adjusted to fall within a range of 12 kPa to 57 kPa (a 90 mmHg to 430 mmHg as reduced to "mmHg") during blowing pressurized steam into the processing container. From the viewpoint of controlling the emulsion temperature to a lower temperature in the deodorization treatment and effectively suppressing generation of aggregates of polymer particles, the pressure in the container is preferably 12 kPa (90 mmHg) to 40 kPa (300 mmHg), more preferably 12 kPa (90 mmHg) to 30 kPa (225 mmHg).

In order to avoid bumping of the emulsion, the contents of the container is preferably stirred during blowing pressurized steam into the container. Since stirring facilitates provision of bubbles, a defoaming agent may be used in an appropriate amount to suppress bubbles. Examples of preferred defoaming agents include a polyether-based defoaming agent (e.g., SN Defoamer PC, product of San Nopco Ltd.) and a silicone-based defoaming agent (e.g., KS-66, product of Shin-Etsu Chemical Co., Ltd.).

The employed pressurized steam preferably has a gage pressure of about 0.05 to about 0.50 MPa (temperature at 110 to 160°C), more preferably a gage pressure of 0.05 to 0.40 MPa, still more preferably a gage pressure of 0.10 to 0.30 MPa. The amount of pressurized steam fed to the processing container is preferably 5 to 100 parts by mass of pressurized steam, with respect to 100 parts by mass of the emulsion, more preferably 5 to 90 parts by mass, still more preferably 5 to 80 parts by mass. When the amount of pressurized steam to satisfy the aforementioned conditions, thermal hysteresis imposed on the emulsion can be suppressed, whereby loss of stability of the emulsion can be suppressed. In addition, the efficiency of removing volatile organic compounds can be fully enhanced.

The time required for steam treatment, which varies depending on the pressurized steam feeding rate and other conditions, is preferably 10 hours or shorter, from the viewpoint of productivity. In the emulsion of the present disclosure, the volatile organic compound level can be reduced to 100 ppm or lower through the steam treatment for 3 to 6 hours.

The non-volatile organic compound concentration of the emulsion of the present disclosure is preferably 30 to 70 mass%, more preferably 40 to 60 mass%. As used herein, the term "non-volatile organic compound concentration" refers to a mass of residue of a sample after heating at 155°C for 30 minutes in a hot air drier, or the ratio of the mass of the residue to the mass of the sample before heating.

The viscosity of the emulsion of the present disclosure, as determined by means of a B-type viscometer at 25°C and 12 rpm, is preferably 10 to 2,000 mPa·s, more preferably 10 to 500 mPa·s, still more preferably 10 to 300 mPa·s. The pH of the emulsion of the present disclosure is preferably 2 to 9.

<Composition of aqueous polymer emulsion for cosmetics>

By incorporating one or more of the below-described components and the like into the emulsion of the present disclosure, aqueous polymer emulsion compositions for cosmetics are produced. So long as the effects of the present disclosure are not impaired, ingredients generally incorporated into cosmetics may be incorporated to complete cosmetic formulations. The aqueous polymer emulsion composition for cosmetics may contain known ingredients such as a thickener, a film-formation aid, a plasticizer, a humectant, a UV-absorber, fatty acid soap, oil and fat, wax, hydrocarbon oil, ester oil, powder, a polymer compound, an antiseptic, an antioxidant, a pH-regulator, a chelating agent, and a coloring material.

Examples of the thickener include an alkali-swelling type thickener, an associable polyurethane, a carboxylvinyl polymer, a thickening polysaccharide, and a clay mineral.

Examples of the film-formation aid and the plasticizer include cellosolves such as methyl cellosolve, ethyl cellosolve, and butyl cellosolve; carbitols such as carbitol, dimethyl carbitol, diethyl carbitol, butyl carbitol, and dibutyl carbitol; carbonates such as ethylene carbonate, diethylene carbonate, and propylene carbonate; acetates such as cellosolve acetate, butyl cellosolve acetate, butyl carbitol acetate, and sucrose acetate; alcohols such as ethanol, propanol, butanol, pentanol, hexanol, benzyl alcohol, and 2-phenylethanol; diols such as ethylene glycol, propylene glycol, butylene glycol, and hexylene glycol; esters such as phthalic acid diester, adipic acid diester, succinic acid diester, sebacic acid diester, and abietic citric acid ester; benzoic acid esters such as sucrose benzoate; and diethylbenzene.

Examples of the humectant include sorbitol, xylitol, propylene glycol, dipropylene glycol, 1,3-butylene glycol, glycerin, diglycerin, polyethylene glycol, hyaluronic acid, chondroitin sulfate, a pyrrolidonecarboxylate salt, and a DL-pyrrolidonecarboxylate salt.

Examples of the UV-absorber include benzophenone derivatives such as 2-hydroxy-4-methoxybenzophenone, 2-hydroxy-4-methoxybenzophenone-5-sulfonic acid, sodium 2-hydroxy-4-methoxybenzophenone-5-sulfoate, dihydromethoxybenzophenone, sodium dihydroxymethoxybenzophenonesulfonate, 2,4-dihydroxybenzophenone, and tetrahydroxybenzophenone; benzoic acid derivatives such as p-aminobenzoic acid, ethyl p-aminobenzoate, glyceryl p-aminobenzoate, amyl p-dimethylaminobenzoate, and octyl p-dimethylaminobenzoate; methoxycinnamic acid derivatives such as ethyl p-methoxycinnamate, isopropyl p-methoxycinnamate, octyl p-methoxycinnamate, 2-ethoxyethyl p-methoxycinnamate, sodium p-methoxycinnamate, potassium p-methoxycinnamate, and glycerin p-methoxycinnamate mono-2-ethylhexanoate; salicylic acid derivatives such as octyl salicylate, phenyl salicylate, homomentyl salicylate, dipropylene glycol salicylate, ethylene glycol salicylate, myristyl salicylate, and methyl salicylate; urocanic acid; ethyl urocanate; ethyl urocanate ester; 4-tert-butyl-4'-methoxybenzoylmethane; 2-(2'-hydroxy-5'-methylphenyl)benzotriazole; 2-phenyl-5-methylbenzoxazole; methyl anthranylate, and 2-ethylhexyl dimethoxybenzylidenedioxoimidazolidinepropionate.

Examples of the fatty acid soap include C6 to 24 higher fatty acid alkali salts. The fatty acid may be saturated or unsaturated, and examples thereof include capric acid, lauric acid, myristic acid, palmitic acid, stearic acid, isostearic acid, arachidic acid, behenic acid, oleic acid, isooleic acid, linoleic acid, linolenic acid, and archidonic acid. No particular limitation is imposed on the alkali for neutralizing the fatty acid, so long as it is used for producing ordinary soap. Examples of the alkali include potassium hydroxide, sodium hydroxide, triethanolamine, N-methyltaurine, and ammonia.

Examples of fat and oil include avocado oil, camellia oil, evening primrose oil, turtle oil, macadamia nut oil, corn oil, mink oil, olive oil, rapeseed oil, egg yolk oil, sesame oil, apricot kernel oil, wheat germ oil, camellia oil, castor oil, linseed oil, safflower oil, cottonseed oil, perilla oil, soy bean oil, peanut oil, tea seed oil, Torreya nucifera oil, rice bran oil, Chinese tung oil, Japanese tung oil, Jojoba oil, germ oil, triglycerin, glycerin trioctanoate, glycerin triisopalmitate, cacao butter, coconut oil, hardened palm oil, palm oil, palm kernel oil, Japan wax kernel oil, hardened oil, hardened castor oil, and polyoxyethylene adducts thereof.

Examples of the wax include spermaceti, bees wax, high oxidation-value bees wax, shellac, mink wax, lanolin, lanolin acetate, liquid lanolin, carnauba wax, candelilla wax, rice bran wax, bran wax, tallow wax, cotton wax, bay berry wax, insect wax, montan wax, schefflera wax, jojoba wax, sugarcane wax, insect wax, isopropyl lanolin fatty acid, hexyl laurate, reduced lanolin, hard lanolin, shellac wax, polyoxyethylene lanolin alcohol ether, polyoxyethylene lanolin alcohol acetate, polyoxyethylene cholesterol ether, lanolin fatty acid polyethylene glycol, and polyoxyethylene hydrogenated lanolin alcohol ether.

Examples of the hydrocarbon oil include oil components such as paraffin, liquid paraffin, ozokerite, squalene, pristane, ceresin, Vaseline, and microcrystalline wax.

Examples of the ester oil include myristate esters such as isopropyl myristate, butyl myristate, myristyl myristate, isocetyl myristate, octyldodecyl myristate, and 2-hexyldecyl myristate; palmitate esters such as isopropyl palmitate, cetyl palmitate, isostearyl palmitate, 2-ethylhexyl palmitate, 2-hexyldecyl palmitate, and 2-heptylundecyl palmitate; stearate esters such as butyl stearate, isocetyl stearate, cholesteryl stearate, isocetyl isostearate, cholesteryl 12-hydroxystrarate, and N-alkylglycol isostearate; laurate esters such as isopropyl laurate and hexyl laurate; linoleate ester such as ethyl linoleate and isopropyl linoleate; octanoate esters such as cetyl octanoate, hexyldecyl dimethyloctanoate, octyldodecyl dimethyloctanoate, and cetyl isootanoate; oleate esters such as decyl oleate and oleyl oleate; sorbitan fatty acid ester oils such as sorbitan monolaurate, sorbitan monopalmitate, sorbitan monostearate, sorbitan sesquistearate, sorbitan tristearate, sorbitan monooleate, sorbitan sesquioleate, sorbitan trioleate, sorbitan monoisostearate, and sorbitan sesquiisostearate; polyoxyethylene sorbitan fatty acid esters such as polyoxyethylene sorbitan monococofatty acid ester, polyoxyethylene sorbitan monopalmitate, polyoxyethylene sorbitan monostearate, polyoxyethylene sorbitan monoisostearate, polyoxyethylene sorbitan tristearate, and polyoxyethylene sorbitan monooleate; polyoxyethylene sorbit fatty acid esters such as polyoxyethylene sorbit monolaurate, polyoxyethylene sorbit hexastearate, polyoxyethylene sorbit tetrastearate, and polyoxyethylene sorbit tetraoleate; lactate esters such as cetyl lactate and myristyl lactate; malate esters such as diisostearyl malate; adipate esters such as diisobutyl adipate, di-2-heptylundecyl adipate, and 2-hexyldecyl adipate; sebacate esters such as di-2-ethylhexyl sebacate and diisopropyl sebacate; succinate esters such as 2-ethylhexyl succinate; citrate esters such as triethyl citrate, triisocetyl citrate, triisoarachyl citrate, triisooctyl citrate, acetyl triethyl citrate, and acetyl tributyl citrate; polyhydric alcohol esters such as ethylene glycol di-2-ethylhexanoate, propylene glycol monocaprate, propylene glycol dicaprate, propylene glycol didecanate, glyceryl tri(2-ethylhexanoate), glyceryl tri(caprylate caprate), trimethylolpropane tri(2-ethylhexanoate), trimethylolpropane triisostearate, penaerythritol tetra(2-ethylhexanoate), glyceryl tri(2-ethylhexanoate), glyceryl trimyristate, decaglyceryl decastearate, decaglyceryl decaoleate, decaglyceryl decaisostearate, glyceryl di-2-heptylundecanate, polyoxyethylene glyceryl monostearate, polyethylene glycol glyceryl monooleate, diglyceryl monostearate, diglyceryl monoisostearate, diglyceryl monooleate, tetraglyceryl monostearate, polyglyceryl monooleate, polyglyceryl tristearate, polyglyceryl pentastearate, polyglyceryl pentaoleate, and neopentyl glycol dicaprate; lanolin acetate; dipenaerythritol fatty acid ester; tri-2-heptylundecanoic acid glyceride; castor oil fatty acid methyl ester; acetoglyceride; 2-octyldodecyl N-lauroyl-L-glutamate; and ethyl laurate.

No particular limitation is imposed on the powder, so long as it can be generally incorporated into a resin for use in cosmetics. Any of the powder may be used, not depending on the morphology of the powder (e.g., spherical, acicular, or plate-like), the particle size (e.g., fume, microparticle, or pigment-level), particle structure (e.g., porous or non-porous), etc.

Examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, synthetic mica, mica, kaolin, sericite, muscovite, phlogopite, lepidolite, biotite, lepidolite, silicic acid, silicic anhydrate, aluminum silicate, magnesium silicate, aluminum magnesium silicate, calcium silicate, barium silicate, strontium silicate, metal tungstate salts, hydroxyapatite, vermiculite, Higilite, bentonite, montmorillonite, hectorite, zeolite, ceramic powder, calcium monohydrogenphosphate, alumina, aluminum hydroxide, boron nitride, boron nitride, and silica.

Examples of the organic powder include starch powder, polyamide powder, polyester powder, polyethylene powder, polypropylene powder, polystyrene powder, polyurethane powder, benzoguanamine powder, polymethylbenzoguanamine powder, tetrafluoroethylene powder, poly(methyl methacrylate) powder, cellulose, silk powder, nylon powder, nylon 12, nylon 6, styrene-acrylic acid copolymer, divinylbenzene-styrene copolymer, vinylic resin, urea resin, phenolic resin, fluororesin, silicon resin, acrylic resin, melamine resin, epoxy resin, polycarbonate resin, microcrystalline fiber powder, rice starch, and lauroyl lysine.

No particular limitation is imposed on the polymer compound, so long as it is generally blended with the resin for cosmetics. The polymer compound can be categorized into a natural polymer compound, a semi-synthesized polymer compound, and synthesized polymer compound.

Examples of the natural polymer compound include gum arabic, tragacanth gum, galactan, guar gum, carob gum, karaya gum, carrageenan, pectin, cannan, quince seed (marmelo), alkecoid (gasso extract), starch (rice, corn, potato, or wheat), glycyrrhizic acid, xanthan gum, dehydroxanthan gum, dextran, succinoglucan, pullulan, collagen, casein, albumin, and gelatin.

Examples of the semi-synthesized polymer compound include starch-based compounds such as carboxymethine starch, methylhydroxypropyl starch, modified potato starch, modified corn starch, and hydroxypropyl starch phosphate; cellulose-based polymers such as methylcellulose, nitrocellulose, ethylcellulose, hydroxypropylmethylcellulose, hydroxyethylcellulose, sodium cellulosesulfate, hydroxypropylcellulose, sodium carboxymethylcellulose (CMC), crystalline cellulose, and cellulose powder; and alginic acid-based polymer such as sodium alginate and propylene glycol alginate.

Examples of the synthesized polymer compound include vinylic polymers such as poly(vinyl alcohol), poly(vinyl methyl ether), polyvinylpyrrolidone, and a carboxyvinyl polymer (Carbopol); polyoxyethylene-based polymers such as polyethylene glycol 2000, 4000, and 6000; polyoxyethylene-polyoxypropylene copolymer-base polymer; acrylic polymers such as vinyl acetate-based polymer, poly(meth)acrylate ester, and polyacrylamide; polyethyleneimine; and cationic polymers. These polymer compound may assume any of liquid, microparticles, particles, etc. Also, the polymer compound may be converted to a dispersion or an emulsion.

Examples of the antiseptic include alkyl p-oxy benzoate, benzoic acid, sodium benzoate, sorbic acid, potassium sorbate, phenoxyethanol, and dehydroacetic acid, and salts thereof.

Examples of the antioxidant include tocopherol, butylhydroxyanisole, and dibutylhydroxytoluene.

Examples of the pH-adjuster include lactic acid, citric acid, glycolic acid, succinic acid, tartaric acid, dl-malic acid, potassium carbonate, sodium hydrogen carbonate, and ammonium hydrogen carbonate.

Examples of the chelating agent include alanine, sodium edetate, sodium polyphosphate, sodium metaphosphate, and phosphoric acid.

<Cosmetic>

The cosmetic of the present disclosure may be used as a makeup cosmetic, a skin cosmetic, a hair cosmetic composition, etc., by incorporating a suitably chosen additional ingredient into the aqueous polymer emulsion. Examples of the makeup cosmetic include eyeliner, eyeshadow, eyeblow, mascara, manicure, cheek rouge, foundation, and lipstick. Examples of the skin cosmetic include pack, sunscreen, milky lotion, cream, and skin lotion. Examples of the hair cosmetic include chemical hair dye, general hair dye, permanent liquid, hair bleach, hair foam, shampoo, and rinse.

Examples

The present disclosure will next be described in detail by way of examples, which should not be construed as limiting the disclosure thereto. Unless otherwise specified, "part(s)" and "%" denote part(s) by mass and mass%, respectively.

1. Production of aqueous polymer emulsions

[Example 1]

Production of aqueous polymer emulsion

<Emulsion polymerization step>

Into a reaction container equipped with a stirrer, a thermometer, a condenser, a nitrogen-introduction pipe, and two dropping funnels, there were fed a monomer (10 parts) having a first-stage composition shown in Table 1, water (45 parts), ethanol (0.0059 parts), and a surfactant (Neopelex G-15; solution of sodium dodecylbenzenesulfonate in water (solid content: 16%), product of Kao Corporation; hereinafter may also be referred to as "G-15") (3.3 parts). The amount of the fed surfactant corresponded to a solid content of 0.53 parts, with respect to the total amount (100 parts by mass) of the monomer having a first-stage and that having a second-stage composition. The contents were heated to 80°C.

Subsequently, G-15 (10 parts), a surfactant (Emulgen 1150S-60; solution of polyoxyethylene alkyl ether in water (solid content: 60%), product of Kao Corporation; hereinafter may also be referred to as "1150S-60") (5 parts), and water (40 parts) were added to a monomer mixture (90 parts) having a second-stage composition shown in Table 1 under mixing, to thereby prepare a monomer pre-emulsion.

To the reaction container, 5% aqueous ammonium persulfate (1 part) was added, and first-stage polymerization reaction was initiated at 80°C under nitrogen. Twenty minutes after initiation of the reaction, where the percent polymerization reached 50 to 100%, the above-prepared monomer pre-emulsion (the entire amount) and 5% aqueous ammonium persulfate serving as a polymerization initiator (20 parts) were continuously added to the reaction container through individual dropping funnels over 4 hours. While the liquid was maintained at about 80°C, second-stage polymerization reaction was conducted. After completion of dropwise addition, the temperature of the liquid was maintained at 80°C for 2 hours. Further, 5% aqueous ammonium persulfate (1 part) was added to the reaction container, and the temperature of the liquid was maintained at 80°C for 1 hour. Thereafter, the contents were cooled to 50°C, to thereby terminate polymerization. The pH of the thus-obtained emulsion was adjusted to 8 with aqueous ammonia, and a defoaming agent (SN Defoamer PC, product of San Nopco Ltd.; hereinafter may also be referred to as "SN Defoamer PC") (0.1 parts) was added thereto, to thereby yield an aqueous polymer emulsion.

<Deodorization step (a step of blowing pressurized steam)>

A pressurized steam supply pipe and a gas exhaust pipe were attached to a flask in which the aqueous polymer emulsion was placed. The temperature of the liquid was elevated to 55°C, and then, pressurized steam (pressure: 0.2 MPa) was blown into the aqueous polymer emulsion (100 parts by mass) at a rate of 5 parts/Hr under stirring, while steam in the system was discharged by means of a vacuum pump through the gas exhaust pipe. As a result, the pressure in the flask was reduced to 15 kPa, and a water boiling state in the system was maintained. By blowing pressurized steam over 5 hours, 25 parts of steam was supplied in total. The pH of the thus-obtained emulsion was adjusted to 8 with aqueous ammonia, to thereby yield an odor-reduced aqueous polymer emulsion.

[Examples 2 to 6, 16 to 20, and 24 to 32]

Production of aqueous polymer emulsions

The procedure of Example 1 was repeated, except that monomer compositions and the amount of G-15 added to the reaction containers were modified as shown in Tables 1 and 2, to thereby yield aqueous polymer emulsions of Examples 2 to 6, 16 to 20, and 24 to 32. Notably, the numerical data given in Tables 1 and 2 without unit refer to those with a unit of "part(s)," and a blank column denotes that no specific item was used. In Tables 1 and 2, in addition to the amount of surfactant (G-15, 1150S-60) fed to the reaction container in the first-stage step, the net amount (solid content) of surfactant present in the reaction container in the first-stage step is shown. "Ethanol concentration in 1st-stage polymerization" given in Tables 1 and 2 were calculated from the total amount of each ingredient fed to the reaction container and the amount of ethanol added in the first-stage polymerization.

[Example 7]

Production of aqueous polymer emulsion

The procedure of Example 1 was repeated, except that the time of supplying pressurized steam in the deodorization step was changed to 3 hours, to thereby yield an aqueous polymer emulsion of Example 7.

[Example 8]

Production of aqueous polymer emulsion

The procedure of Example 1 was repeated, except that the pressure in the container was changed to 20 kPa (water non-boiling state) in the deodorization step, to thereby yield an aqueous polymer emulsion of Example 8.

[Example 9]

Production of aqueous polymer emulsion

The procedure of Example 1 was repeated, except that no deodorization step was performed, to thereby yield an aqueous polymer emulsion of Example 9.

[Example 10]

Production of aqueous polymer emulsion

The procedure of Example 1 was repeated, except that no ethanol was added, to thereby yield an aqueous polymer emulsion of Example 10.

[Example 11]

Production of aqueous polymer emulsion

The procedure of Example 1 was repeated, except that the amount of ethanol added was changed to 0.00059 parts, to thereby yield an aqueous polymer emulsion of Example 11.

[Example 12]

Production of aqueous polymer emulsion

The procedure of Example 1 was repeated, except that the amount of ethanol added was changed to 0.0018 parts, to thereby yield an aqueous polymer emulsion of Example 12.

[Example 13]

Production of aqueous polymer emulsion

The procedure of Example 1 was repeated, except that the amount of ethanol added was changed to 0.030 parts, to thereby yield an aqueous polymer emulsion of Example 13.

[Example 14]

Production of aqueous polymer emulsion

The procedure of Example 1 was repeated, except that the amount of ethanol added was changed to 0.18 parts, to thereby yield an aqueous polymer emulsion of Example 14.

[Example 15]

Production of aqueous polymer emulsion

The procedure of Example 1 was repeated, except that the amount of ethanol added was changed to 0.59 parts, to thereby yield an aqueous polymer emulsion of Example 15.

[Example 21]

Production of aqueous polymer emulsion

<Emulsion polymerization step>

Into a reaction container equipped with a stirrer, a thermometer, a condenser, a nitrogen-introduction pipe, and two dropping funnels, there were fed a monomer (10 parts) having a first-stage composition shown in Table 2, water (45 parts), ethanol (0.0059 parts), and a surfactant (G-15) (3.3 parts). The contents were heated to 80°C.

Subsequently, G-15 (8 parts) and 1150S-60 (5 parts) both serving as surfactants, and water (40 parts) were added to a monomer mixture (90 parts) having a second-stage composition shown in Table 2 under mixing, to thereby prepare a monomer pre-emulsion.

To the reaction container, 5% aqueous ammonium persulfate (1 part) was added, and first-stage polymerization reaction was initiated at 80°C under nitrogen. Twenty minutes after initiation of the reaction, where the percent polymerization reached 50 to 100%, the above-prepared monomer pre-emulsion (the entire amount) and 5% aqueous ammonium persulfate serving as a polymerization initiator (20 parts) were continuously added to the reaction container through individual dropping funnels over 4 hours. While the liquid was maintained at about 80°C, second-stage polymerization reaction was conducted. From start of dropwise addition, G-15 (1 part) was added at intervals of 1 hour (twice in total) to the reaction container. After completion of dropwise addition, the temperature of the liquid was maintained at 80°C for 2 hours. Further, 5% aqueous ammonium persulfate (1 part) was added to the reaction container, and the temperature of the liquid was maintained at 80°C for 1 hour. Thereafter, the contents were cooled to 50°C, to thereby terminate polymerization. The pH of the thus-obtained emulsion was adjusted to 8 with aqueous ammonia, and SN Defoamer PC serving as a defoaming agent (0.1 parts) was added thereto, to thereby yield an aqueous polymer emulsion.

<Deodorization step>

The procedure of Example 1 was repeated to perform the deodorization treatment for reducing mal-odor, to thereby yield an aqueous polymer emulsion of Example 21.

[Example 22]

Production of aqueous polymer emulsion

The procedure of Example 21 was repeated, except that the amount of surfactant G-15 used in preparation of a monomer pre-emulsion was changed to 7 parts, and addition of G-15 after initiation of dropwise addition of the monomer pre-emulsion and the polymerization initiator to the reaction container was conducted in such a manner that G-15 (0.75 parts) was added with intervals of 30 minutes (4 times in total) from initiation of dropwise addition of the monomer pre-emulsion and the polymerization initiator, to thereby yield an aqueous polymer emulsion of Example 22.

[Example 23]

Production of aqueous polymer emulsion

The procedure of Example 21 was repeated, except that the amount of surfactant G-15 used in preparation of a monomer pre-emulsion was changed to 7 parts, and addition of G-15 after initiation of dropwise addition of the monomer pre-emulsion and the polymerization initiator to the reaction container was conducted in such a manner that G-15 (0.5 parts) was added with intervals of 30 minutes (6 times in total) from initiation of dropwise addition of the monomer pre-emulsion and the polymerization initiator, to thereby yield an aqueous polymer emulsion of Example 23.

[Example 33]

Production of aqueous polymer emulsion

<Emulsion polymerization step>

Into a reaction container equipped with a stirrer, a thermometer, a condenser, a nitrogen-introduction pipe, and two dropping funnels, there were fed water (45 parts), ethanol (0.0059 parts), and G-15 serving as a surfactant (2.5 parts). The contents were heated to 80°C.

Subsequently, G-15 (10 parts) and 1150S-60 (5 parts) serving as surfactants and water (40 parts) were added to a monomer mixture of n-butyl methacrylate (BMA) originating from petroleum (i.e., non-biomass origin) (60 parts), n-butyl acrylate (BA) originating from petroleum (i.e., non-biomass origin) (35 parts), and methacrylic acid (MAA) originating from petroleum (i.e., non-biomass origin) (5 parts) under mixing, to thereby prepare a monomer pre-emulsion.

A 1% aliquot of the thus-prepared monomer pre-emulsion (1.55 parts) was added to the reaction container, and stirring was conducted for 5 minutes. Then, 5% aqueous ammonium persulfate (1 part) was added, and first-stage polymerization reaction was initiated at 80°C under nitrogen. Twenty minutes after initiation of the reaction, where the percent polymerization reached 50 to 100%, the remaining portion (99%) of the above-prepared monomer pre-emulsion and 5% aqueous ammonium persulfate serving as a polymerization initiator (20 parts) were continuously added to the reaction container through individual dropping funnels over 4 hours. While the liquid was maintained at about 80°C, second-stage polymerization reaction was conducted. After completion of dropwise addition, the temperature of the liquid was maintained at 80°C for 2 hours. Further, 5% aqueous ammonium persulfate (1 part) was added to the reaction container, and the temperature of the liquid was maintained at 80°C for 1 hour. Thereafter, the contents were cooled to 50°C, to thereby terminate polymerization. The pH of the thus-obtained emulsion was adjusted to 8 with aqueous ammonia, and SN Defoamer PC serving as a defoaming agent (0.1 parts) was added thereto, to thereby yield an aqueous polymer emulsion.

<Deodorization step>

The procedure of Example 1 was repeated to perform the deodorization treatment for reducing mal-odor, to thereby yield an aqueous polymer emulsion of Example 33.

[Comparative Examples 1 to 4]

Production of aqueous polymer emulsions

The procedure of Example 1 was repeated, except that the monomer composition and the amount of G-15 fed to the reaction container shown in Table 2 were modified, to thereby yield aqueous polymer emulsions of Comparative Examples 1 to 4.

[Comparative Example 5]

Production of aqueous polymer emulsion

The procedure of Example 1 was repeated, except that the amount of ethanol added was changed to 2 parts, to thereby yield an aqueous polymer emulsion of Comparative Example 5.

[Comparative Example 6]

Production of aqueous polymer emulsion

<Emulsion polymerization step>

Into a reaction container equipped with a stirrer, a thermometer, a condenser, a nitrogen-introduction pipe, and two dropping funnels, there were fed a monomer (10 parts) having a first-stage composition shown in Table 2, water (45 parts), and 1150S-60 serving as a surfactant (0.8 parts). The contents were heated to 80°C.
Subsequently, 1150S-60 (7.7 parts) serving as a surfactant and water (52 parts) were added to a monomer mixture (90 parts) having a second-stage composition shown in Table 2 under mixing, to thereby prepare a monomer pre-emulsion.
To the reaction container, 5% aqueous ammonium persulfate (1 part) was added, and first-stage polymerization reaction was initiated at 80°C under nitrogen. Twenty minutes after initiation of the reaction, where the percent polymerization reached 50 to 100%, the above-prepared monomer pre-emulsion (the entire amount) and 5% aqueous ammonium persulfate serving as a polymerization initiator (20 parts) were continuously added to the reaction container through individual dropping funnels over 4 hours. While the liquid was maintained at about 80°C, second-stage polymerization reaction was conducted. After completion of dropwise addition, the temperature of the liquid was maintained at 80°C for 2 hours. Further, 5% aqueous ammonium persulfate (1 part) was added to the reaction container, and the temperature of the liquid was maintained at 80°C for 1 hour. Thereafter, the contents were cooled to 50°C, to thereby terminate polymerization. The pH of the thus-obtained emulsion was adjusted to 8 with aqueous ammonia, and SN Defoamer PC serving as a defoaming agent (0.1 parts) was added thereto, to thereby yield an aqueous polymer emulsion.

<Deodorization step>

The procedure of Example 1 was repeated to perform the deodorization treatment for reducing mal-odor, to thereby yield an aqueous polymer emulsion of Comparative Example 6.

2. Evaluation

(1) Evaluation of aqueous polymer emulsions

The liquid properties and physical properties of the aqueous polymer emulsions of Examples 1 to 33 and Comparative Examples 1 to 6 were evaluated in the following manner.

<Non-volatile component concentration>

An aqueous polymer emulsion was heated at 155°C for 30 minutes by means of a hot air drier. The ratio of the residue was calculated as non-volatile component concentration of the aqueous polymer emulsion by the following numerical expression:

$$\text{Non-volatile component concentration (\%)} = (W3/W2) \times 100$$

(wherein W2 represents a mass of emulsion (unit: g) before heating, and W3 represents a mass of residual component (unit: g) after heating).

<Viscosity>

Measured by means of a B-type viscometer at 25°C and 12 rpm.

<Volume average particle size, cumulative average particle size, and particle size distribution factor of aqueous polymer emulsion>

The volume average particle size, cumulative average particle size, and particle size distribution factor (volume average particle size/cumulative average particle size) were measured by means of a particle size analyzer (UPA-EX150, product of MicrotracBEL Corp.).

<Glass transition temperature of polymer>

The glass transition temperature (Tg) of a polymer was calculated by the aforementioned numerical expression (2).

<Water resistance>

Water resistance of the aqueous polymer emulsion after drying was evaluated through the following procedure. Firstly, an aqueous polymer emulsion was applied onto a glass plate by means of an applicator so as to have a dry thickness of 50 μm, and air-dried at 23°C and 50%RH for 1 day, to thereby prepare a test piece. The thus-prepared test piece was immersed in distilled water for 1 day and rubbed with a water-absorbed sponge of a wear tester at a load of 100 g and 10 reciprocated rubbing motions. Occurrence of falling and peeling were evaluated on the basis of the following ratings. ⊚: No change was observed in the coating film (good)

O: Slight peeling or falling was observed in the coating film (fair)
Δ: Peeling or falling was observed in about a half portion of the coating film (slightly bad)
X: Peeling or falling was observed in a considerable portion of the coating film (bad)

<Oil resistance>

A test piece prepared through the same method as employed in the aforementioned water resistance test was immersed in oleic acid for 1 day. The test piece was rubbed with an oleic acid-absorbed sponge of a wear tester at a load of 100 g and 10 reciprocated rubbing motions, and occurrence of falling and peeling were evaluated on the basis of the same ratings as employed in the aforementioned water resistance test.

<Softness>

An aqueous polymer emulsion was applied onto a polyethylene terephthalate film (thickness: 25 μm) so as to have a dry thickness of 25 μm and then, dried by means of a hot air drier at 50°C for 6 hours. The dried piece was air-dried at 23°C and 50%RH for 1 day, to thereby prepare a test piece formed of a polyethylene terephthalate film with a coating film formed thereon. The thus-obtained test piece was subjected to bending action 10 times, and cracking or falling of the coating film was evaluated on the basis of the following ratings.

O: No change was observed in the coating film (good)
Δ: Slight cracking or falling was observed in the coating film (slightly bad)
X: Peeling or falling was observed in the entire surface of the coating film (bad)

<Tackiness>

The surface of a test piece was traced with a finger the test piece having been prepared through the same method as employed in the aforementioned softness test. The stickiness of the coating film was determined through sensation of touch and evaluated on the basis of the following ratings. The less the stickiness, the more suitable the sensation in use.

◎: No stickiness (good)
O: Slightly sticky (fair)
Δ: Sticky (slightly bad)
X: Very sticky (bad)

<Stability over time>

The stability over time of an aqueous polymer emulsion was evaluated through the following procedure. Firstly, the volume average particle size of an as-produced aqueous polymer emulsion was measured. Then, the emulsion was placed in a container, and the container was tightly closed. The container was maintained in a hot air drier at 40°C for 1 month. Thereafter, the volume average particle size was measured again. The volume average particle size was measured by means of a particle size analyzer (UPA-EX150, product of MicrotracBEL Corp.). From the volume average particle size of the as-produced aqueous polymer emulsion (i.e., an initial particle size) and the volume average particle size of the as-produced aqueous polymer emulsion after maintenance in a hot air drier at 40°C for 1 month (i.e., a 1-month particle size), a percent particle size change (%) was calculated by the following numerical expression, and the stability over time of the aqueous polymer emulsion was evaluated on the basis of the following ratings.

Percent particle size change (%) = [(initial particle size - 1-month particle size)/initial particle size]×100

◎: Percent particle size change of <5% (very good)
O: Percent particle size change of ≥5% and <10% (fair)
Δ: Percent particle size change of ≥10% and <15% (slightly bad)
X: Percent particle size change of ≥15% (bad)

<Evaluation of odor originating from volatile organic compound>

An aqueous polymer emulsion (80 g) was placed in a glass container (100 mL) and closely sealed in the container with a cap. The container was maintained in a hot air drier at 40°C for 1 hour and the immediately removed from the drier. Opening the cap, the odor was evaluated through a sensory test, and the odor level was evaluated on the basis of the following ratings.

◎: Substantially no odor (very good)
O: Slight odor (good)
Δ: Odor (slightly bad)
X: Strong odor (bad)

<Volatile organic compound level>

The volatile organic compound (e.g., an unreacted monomer) content was measured by means of a gas chromatograph (apparatus: GC-2014 (product of Shimadzu Corporation), column: DB-1 (product of GL Science), carrier gas: nitrogen, and detector: hydrogen flame ionization detector). In gas chromatographic analysis, an aqueous polymer emulsion (0.5 g), ethanol (4.0 g), and 20% aqueous calcium chloride (0.3 g) were mixed, and the mixture was allowed to stand for 10 minutes. The mixture was subjected to centrifugation at 12,000 rpm for 10 minutes, and the supernatant (1.5 g) was taken, to thereby provide an internal standard. A 1% ethylene glycol monomethyl ether acetate aqueous solution (0.05 g) was added thereto, to thereby provide an injection liquid sample of gas chromatography.

Each of the aqueous polymer emulsions of Examples 1 to 33 and Comparative Examples 1 to 6 were evaluated in terms of liquid property, water resistance, oil resistance, softness, tackiness, stability over time, odor of volatile organic compound, and volatile organic compound level. Tables 3 and 4 show the results.

[Table 1]

|  | Examples | | | | | | | | | | | | | | | | | | |
|---|---|---|---|---|---|---|---|---|---|---|---|---|---|---|---|---|---|---|---|
|  | 1 | 2 | 3 | 4 | 5 | 6 | 7 | 8 | 9 | 10 | 11 | 12 | 13 | 14 | 15 | 16 | 17 | 18 | 19 |
| Amount of G-15 (solid content: 16%) fed to reaction container in 1st-stage step | 3.3 | 3.3 | 3.3 | 3.3 | 3.3 | 3.3 | 3.3 | 3.3 | 3.3 | 3.3 | 3.3 | 3.3 | 3.3 | 3.3 | 3.3 | 3.3 | 3.3 | 4.7 | 1.3 |
| Net amount of G-15 fed to reaction container in 1st-stage step | 0.53 | 0.53 | 0.53 | 0.53 | 0.53 | 0.53 | 0.53 | 0.53 | 0.53 | 0.53 | 0.53 | 0.53 | 0.53 | 0.53 | 0.53 | 0.53 | 0.53 | 0.75 | 0.21 |
| No. of G-15 addition operations separate from dropwise supply of monomer emulsion | | | | | | | | | | | | | | | | | | | |
| Amount of 1150S-60 (solid content: 60%) fed to reaction container in 1st-stage step | | | | | | | | | | | | | | | | | | | |
| Net amount of 1150S-60 fed to reaction container in 1st-stage step | | | | | | | | | | | | | | | | | | | |
| Monomers 1st Stage A IBMA | | | | | | | | | | | | | | | | | | | |
| Monomers 1st Stage A BMA | 10 | 10 | 10 | 10 | 10 | 10 | 10 | 10 | 10 | 10 | 10 | 10 | 10 | 10 | 10 | 2 | 5 | 10 | 10 |
| Monomers 1st Stage A BA | | | | | | | | | | | | | | | | | | | |
| Monomers 1st Stage A 2EHA | | | | | | | | | | | | | | | | | | | |
| Monomers 1st Stage B MAA | | | | | | | | | | | | | | | | | | | |
| Monomers 1st Stage B AA | | | | | | | | | | | | | | | | | | | |
| Subtotal | 10 | 10 | 10 | 10 | 10 | 10 | 10 | 10 | 10 | 10 | 10 | 10 | 10 | 10 | 10 | 2 | 5 | 10 | 10 |
| Monomers 2nd Stage A IBMA | | | | | | | | | | | | | | | | | | | |
| Monomers 2nd Stage A BMA | 50 | 52 | 57 | 57.5 | 49 | 40 | 50 | 50 | 50 | 50 | 50 | 50 | 50 | 50 | 50 | 59 | 56 | 50 | 50 |
| Monomers 2nd Stage A BA | 35 | 35 | 32 | 32 | 35 | 40 | 35 | 35 | 35 | 35 | 35 | 35 | 35 | 35 | 35 | 35 | 35 | 35 | 35 |
| Monomers 2nd Stage A 2EHA | | | | | | | | | | | | | | | | | | | |
| Monomers 2nd Stage B MAA | 5 | 3 | 1 | 0.5 | 6 | 10 | 5 | 5 | 5 | 5 | 5 | 5 | 5 | 5 | 5 | 4 | 4 | 5 | 5 |
| Monomers 2nd Stage B AA | | | | | | | | | | | | | | | | | | | |
| Monomers 2nd Stage Additional LA | | | | | | | | | | | | | | | | | | | |
| Monomers 2nd Stage Additional MA | | | | | | | | | | | | | | | | | | | |
| Monomers 2nd Stage Additional St | | | | | | | | | | | | | | | | | | | |
| Subtotal | 90 | 90 | 90 | 90 | 90 | 90 | 90 | 90 | 90 | 90 | 90 | 90 | 90 | 90 | 90 | 98 | 95 | 90 | 90 |
| Tg [°C] of polymer | -6.7 | -8.4 | -7.7 | -8.2 | -6.7 | -6.4 | -6.7 | -6.7 | -6.7 | -6.7 | -6.7 | -6.7 | -6.7 | -6.7 | -6.7 | -7.6 | -7.6 | -7.6 | -7.6 |
| Ethanol concentration [ppm] in 1st-stage polymerization | 100 | 100 | 100 | 100 | 100 | 100 | 100 | 100 | 100 | 0 | 10 | 30 | 500 | 2,982 | 9,804 | 116 | 109 | 98 | 103 |
| Deodorization step | Yes | Yes | Yes | Yes | Yes | Yes | Yes | Yes | No | Yes | Yes | Yes | Yes | Yes | Yes | Yes | Yes | Yes | Yes |
| Temp. [°C] in container during deodorization | 55 | 55 | 55 | 55 | 55 | 55 | 55 | 55 | - | 55 | 55 | 55 | 55 | 55 | 55 | 55 | 55 | 55 | 55 |
| Pressure [kPa] in container during deodorization | 15 | 15 | 15 | 15 | 15 | 15 | 15 | 20 | - | 15 | 15 | 15 | 15 | 15 | 15 | 15 | 15 | 15 | 15 |
| Time [Hr] of pressurized steam supply | 5 | 5 | 5 | 5 | 5 | 5 | 3 | 5 | - | 5 | 5 | 5 | 5 | 5 | 5 | 5 | 5 | 5 | 5 |

EP 4 609 849 A1

[Table 2]

| | | | Examples | | | | | | | | | | | | | | | Comparative Examples | | | | | |
|---|---|---|---|---|---|---|---|---|---|---|---|---|---|---|---|---|---|---|---|---|---|---|---|---|
| | | | 20 | 21 | 22 | 23 | 24 | 25 | 26 | 27 | 28 | 29 | 30 | 31 | 32 | 33 | 1 | 2 | 3 | 4 | 5 | 6 |
| Amount of G-15 (solid content: 16%) fed to reaction container in 1st-stage step | | | 1.1 | 3.3 | 3.3 | 3.3 | 3.3 | 3.3 | 3.3 | 3.3 | 3.3 | 3.3 | 3.3 | 3.3 | 3.3 | 2.5 | 3.3 | 3.3 | 5.3 | 0.6 | 3.3 | |
| Net amount of G-15 fed to reaction container in 1st-stage step | | | 0.18 | 0.53 | 0.53 | 0.53 | 0.53 | 0.53 | 0.53 | 0.53 | 0.53 | 0.53 | 0.53 | 0.53 | 0.53 | 0.40 | 0.53 | 0.53 | 0.85 | 0.10 | 0.53 | |
| No. of G-15 addition operations separate from dropwise supply of monomer emulsion | | | | 2 | 4 | 6 | | | | | | | | | | | | | | | | |
| Amount of 1150S-60 (solid content: 60%) fed to reaction container in 1st-stage step | | | | | | | | | | | | | | | | | | | | | | 0.8 |
| Net amount of 1150S-60 fed to reaction container in 1st-stage step | | | | | | | | | | | | | | | | | | | | | | 0.48 |
| Monomers | 1st Stage | A IBMA | 10 | 10 | 10 | 10 | 10 | 10 | | 10 | 10 | 10 | 10 | 10 | 10 | 0.60 | 10 | 10 | 10 | 10 | 10 | 10 |
| | | A BMA | | | | | | | | | | | | | | | | | | | | |
| | | A BA | | | | | | | | | | | | | | | | | | | | |
| | | A 2EHA | | | | | | | 10 | | | | | | | | | | | | | |
| | | B MAA | 10 | 10 | 10 | 10 | 10 | 10 | 10 | 10 | 10 | 10 | 10 | 10 | 10 | 0.35 | 10 | 10 | 10 | 10 | 10 | 10 |
| | | B AA | | | | | | | | | | | | | | 0.05 | | | | | | |
| | | Subtotal | 10 | 10 | 10 | 10 | 10 | 10 | 10 | 10 | 10 | 10 | 10 | 10 | 10 | 1.0 | 10 | 10 | 10 | 10 | 10 | 10 |
| | 2nd Stage | A IBMA | | | | | | | | | | | | | | | | | | | | |
| | | A BMA | 50 | 50 | 50 | 50 | 75 | 83 | 40 | 73 | 57 | 35 | 40 | 40 | 35 | 59.40 | 30 | 35 | 50 | 50 | 50 | 50 |
| | | A BA | 35 | 35 | 35 | 35 | 10 | 2 | 45 | 15 | | 40 | 35 | 25 | 20 | 34.65 | 15 | 43 | 35 | 35 | 35 | 35 |
| | | A 2EHA | | | | | | | | | 28 | | | | | | | | | | | |
| | | B MAA | 5 | 5 | 5 | 5 | 5 | 5 | 5 | | 5 | 5 | 5 | 5 | 5 | 4.95 | 5 | 12 | 5 | 5 | 5 | 5 |
| | | B AA | | | | | | | | 2 | | | | | | | | | | | | |
| | | Additional LA | | | | | | | | | | | | 20 | 30 | | 40 | | | | | |
| | | Additional MA | | | | | | | | | | 10 | 10 | | | | | | | | | |
| | | Additional St | | | | | | | | | | | | | | | | | | | | |
| | | Subtotal | 90 | 90 | 90 | 90 | 90 | 90 | 90 | 90 | 90 | 90 | 90 | 90 | 90 | 99.0 | 90 | 90 | 90 | 90 | 90 | 90 |
| Tg [°C] of polymer | | | -7.6 | -7.6 | -7.6 | -7.6 | 15.4 | 21.4 | -12.3 | 8.0 | -8.1 | -5.6 | -7.6 | -6.8 | -6.9 | -6.9 | -7.0 | -7.1 | -6.7 | -6.7 | -6.7 | -6.7 |
| Ethanol concentration [ppm] in 1st-stage polymerization | | | 104 | 100 | 100 | 100 | 100 | 100 | 100 | 100 | 100 | 100 | 100 | 100 | 100 | 118 | 100 | 100 | 97 | 105 | 32,626 | 0 |
| Deodorization step | | | Yes | Yes | Yes | Yes | Yes | Yes | Yes | Yes | Yes | Yes | Yes | Yes | Yes | Yes | Yes | Yes | Yes | Yes | Yes | Yes |
| Temp. [°C] in container during deodorization | | | 55 | 55 | 55 | 55 | 55 | 55 | 55 | 55 | 55 | 55 | 55 | 55 | 55 | 55 | 55 | 55 | 55 | 55 | 55 | 55 |
| Pressure [kPa] in container during deodorization | | | 15 | 15 | 15 | 15 | 15 | 15 | 15 | 15 | 15 | 15 | 15 | 15 | 15 | 15 | 15 | 15 | 15 | 15 | 15 | 15 |
| Time [Hr] of pressurized steam supply | | | 5 | 5 | 5 | 5 | 5 | 5 | 5 | 5 | 5 | 5 | 5 | 5 | 5 | 5 | 5 | 5 | 5 | 5 | 5 | 5 |

[Table 3]

EP 4 609 849 A1

| | | | Examples | | | | | | | | | | | | | | | | | | |
|---|---|---|---|---|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 14 | 15 | 16 | 17 | 18 | 19 |
| Evaluation | Liq. Properties | Non-volatile component concentration [%] | 46.2 | 46.1 | 45.7 | 46.1 | 46.2 | 46.0 | 46.3 | 46.2 | 46.1 | 46.3 | 46.1 | 46.3 | 46.0 | 46.0 | 46.0 | 46.2 | 46.1 | 46.0 | 45.8 |
| | | Viscosity [mPa.s] | 43 | 30 | 28 | 42 | 67 | 110 | 44 | 42 | 40 | 44 | 40 | 42 | 39 | 39 | 39 | 85 | 44 | 207 | 38 |
| | | pH | 8.0 | 8.0 | 8.0 | 8.0 | 8.0 | 8.0 | 8.0 | 8.0 | 8.0 | 8.0 | 8.0 | 8.0 | 8.0 | 8.0 | 8.0 | 7.9 | 7.9 | 8.0 | 8.0 |
| | | Volume av. particle size[nm] | 113 | 114 | 112 | 114 | 113 | 115 | 113 | 113 | 113 | 113 | 116 | 120 | 121 | 122 | 124 | 75 | 88 | 72 | 132 |
| | | Cumulative av. particle size[nm] | 110 | 112 | 111 | 110 | 109 | 108 | 110 | 110 | 110 | 101 | 110 | 117 | 117 | 116 | 114 | 71 | 87 | 68 | 127 |
| | | Particle size distribution | 1.03 | 1.02 | 1.01 | 1.04 | 1.04 | 1.06 | 1.03 | 1.03 | 1.03 | 1.12 | 1.05 | 1.03 | 1.03 | 1.05 | 1.09 | 1.06 | 1.01 | 1.06 | 1.04 |
| | Phys. Properties | Water resistance | ◎ | ◎ | ◎ | ◎ | ◎ | ○ | ◎ | ◎ | ◎ | ○ | ◎ | ◎ | ◎ | ◎ | ◎ | ◎ | ◎ | ◎ | ○ |
| | | Oil resistance | ◎ | ◎ | ○ | ○ | ◎ | ◎ | ◎ | ◎ | ◎ | ◎ | ◎ | ◎ | ◎ | ◎ | ◎ | ◎ | ◎ | ◎ | ◎ |
| | | Softness | ○ | ○ | ○ | ○ | ○ | △ | ○ | ○ | ○ | ○ | ○ | ○ | ○ | ○ | ○ | ○ | ○ | ○ | ○ |
| | | Tackiness | ◎ | ◎ | ◎ | ○ | ◎ | ◎ | ◎ | ◎ | ◎ | ◎ | ◎ | ◎ | ◎ | ◎ | ◎ | ◎ | ◎ | ◎ | ◎ |
| | | Stability over time | ◎ | ◎ | ◎ | ○ | ◎ | ○ | ◎ | ◎ | ◎ | ◎ | ◎ | ◎ | ◎ | ◎ | ◎ | ◎ | ◎ | ◎ | ◎ |
| | | Odor from volatile organic compound | ○ | ○ | ○ | ○ | ○ | ○ | ○ | △ | △ | ○ | ○ | ○ | ○ | ○ | ○ | ○ | ○ | ○ | ○ |
| | | Volatile organic compound level [ppm] | 4 | 2 | 2 | 1 | 3 | 2 | 48 | 176 | 197 | 2 | 4 | 2 | 1 | 5 | 7 | 2 | 5 | 4 | 3 |

[Table 4]

Abbreviations in the tables are as follows.

| | | | Examples | | | | | | | | | | | | | | Comparative Examples | | | | | |
|---|---|---|---|---|---|---|---|---|---|---|---|---|---|---|---|---|---|---|---|---|---|---|---|
| | | | 20 | 21 | 22 | 23 | 24 | 25 | 26 | 27 | 28 | 29 | 30 | 31 | 32 | 33 | 1 | 2 | 3 | 4 | 5 | 6 |
| Evalu ation | Liq. Prope rties | Non-volatile component concentration [%] | 46.0 | 45.9 | 45.8 | 46.0 | 45.9 | 45.8 | 45.9 | 45.7 | 45.9 | 45.7 | 46.0 | 46.1 | 46.2 | 45.9 | 46.0 | 46.2 | 45.9 | 45.7 | 45.9 | 46.1 |
| | | Viscosity [mPa.s] | 27 | 35 | 34 | 31 | 38 | 35 | 36 | 35 | 36 | 40 | 54 | 42 | 40 | 40 | 41 | 38 | 584 | 29 | 26 | 120 |
| | | pH | 8.0 | 8.0 | 8.0 | 8.0 | 8.0 | 8.0 | 8.0 | 8.0 | 7.9 | 8.0 | 7.9 | 8.0 | 8.0 | 8.0 | 8.0 | 7.9 | 8.0 | 8.0 | 8.0 | 8.0 |
| | | Volume av. particle size[nm] | 148 | 122 | 121 | 129 | 107 | 109 | 108 | 105 | 110 | 105 | 107 | 112 | 117 | 100 | 109 | 112 | 60 | 168 | 167 | 353 |
| | | Cumulative av. particle size[nm] | 138 | 111 | 103 | 100 | 104 | 105 | 107 | 102 | 108 | 100 | 105 | 110 | 115 | 98 | 105 | 108 | 55 | 154 | 124 | 342 |
| | | Particle size distribution | 1.07 | 1.10 | 1.17 | 1.29 | 1.03 | 1.04 | 1.01 | 1.03 | 1.02 | 1.05 | 1.02 | 1.02 | 1.02 | 1.02 | 1.04 | 1.04 | 1.09 | 1.09 | 1.35 | 1.03 |
| | Phys. Prope rties | Water resistance | O | O | O | △ | O | O | ◎ | ◎ | ◎ | ◎ | O | ◎ | ◎ | ◎ | ◎ | × | △ | △ | × | × |
| | | Oil resistance | O | ◎ | O | O | ◎ | O | ◎ | ◎ | ◎ | O | ◎ | O | △ | ◎ | × | ◎ | ◎ | × | × | × |
| | | Softness | O | O | O | O | △ | △ | O | O | O | O | O | O | O | O | O | △ | O | O | O | O |
| | | Tackiness | ◎ | ◎ | ◎ | ◎ | ◎ | ◎ | △ | ◎ | ◎ | ◎ | ◎ | ◎ | ◎ | ◎ | ◎ | ◎ | ◎ | ◎ | ◎ | ◎ |
| | | Stability over time | ◎ | ◎ | ◎ | ◎ | ◎ | ◎ | ◎ | ◎ | ◎ | ◎ | ◎ | ◎ | ◎ | ◎ | ◎ | △ | ◎ | ◎ | ◎ | ◎ |
| | | Odor from volatile organic compound | O | O | O | O | O | O | O | O | O | O | O | O | O | O | O | O | O | O | O | O |
| | | Volatile organic compound level [ppm] | 4 | 1 | 5 | 2 | 2 | 2 | 4 | 6 | 8 | 12 | 2 | 16 | 19 | 2 | 2 | 4 | 2 | 4 | 7 | 10 |

<Monomers>

IBMA: i-butyl methacrylate derived from petroleum (i.e., non-biomass origin)
BMA: n-butyl methacrylate derived from petroleum (i.e., non-biomass origin)
MA: methyl acrylate derived from petroleum (i.e., non-biomass origin)
BA: n-butyl acrylate derived from petroleum (i.e., non-biomass origin)
2EHA: 2-ethylhexyl acrylate derived from petroleum (i.e., non-biomass origin)
St: styrene derived from petroleum (i.e., non-biomass origin) MAA: methacrylic acid derived from petroleum (i.e., non-biomass origin)
AA: acrylic acid derived from petroleum (i.e., non-biomass origin)
LA: biomass-origin lauryl acrylate having a C12 linear-chain alkyl group at an ester end; Biomass acrylate LA (C12 acrylate) (product of Osaka Organic Chemical Industry Ltd.) <Surfactants>
G-15: Neopelex G-15, sodium dodecylbenzenesulfonate (product of Kao Corporation)
1150S-60: Emulgen 1150S-60, polyoxyethylene alkyl ether (product of Kao Corporation)

As shown in Tables 3 and 4, the aqueous polymer emulsions of Examples 1 to 33 were found to exhibit suitable water resistance, oil resistance, and softness in a well-balanced manner, showing excellent performance, as compared with Comparative Example 1 (the composition of monomer (A) falling outside the scope of the present disclosure); Comparative Example 2 (the composition of monomer (B) falling outside the scope of the present disclosure); Comparative Example 3 (a volume average particle size less than 70 nm); Comparative Examples 4 and 6 (a volume average particle size in excess of 150 nm); and Comparative Example 5 (a particle size distribution factor greater than 1.3 and a volume average particle size in excess of 150 nm). In addition, the aqueous polymer emulsions of Examples 1 to 33 were also found to exhibit excellent results in terms of tackiness, stability over time, and low-odor performance.

(2) Production and evaluation of cosmetic compositions

[Example 34]

Production of mascara

To ion-exchanged water (22.3 parts), methylhydroxypropylcellulose (2 parts), talc (10 parts) and the aqueous polymer emulsion obtained in Example 1 (45 parts) were added, and the mixture was uniformly stirred. Subsequently, to the thus-obtained liquid mixture, a color paste containing black iron oxide (15 parts), glycerin (5 parts), and polyoxyethylene sorbitan monooleate (1.5 parts) was added, and the mixture was uniformly mixed through stirring. A perfume (0.1 parts) and an antiseptic (0.1 parts) were added thereto, to thereby yield a black mascara.

[Examples 35 to 50 and Comparative Examples 7 to 12]

Production of mascaras

The procedure of Example 34 was repeated, except that the aqueous polymer emulsion was changed to a corresponding emulsion that shown in Table 5, to thereby yield mascaras of Examples 35 to 50 and Comparative Examples 7 to 12. Notably, the numerical data given in Table 5 without unit refer to those with a unit of "part(s)", and a blank column denotes that no specific item was used (the same applies to Tables 6 to 8).

[Evaluation of mascaras]

Each of the cosmetic compositions (mascaras) of Examples 34 to 50 and Comparative Examples 7 to 12 were evaluated in terms of water resistance, oil resistance, cosmetic effect persistency, and stability over time through the following procedure.

<Water resistance>

A cosmetic composition (mascara) was applied onto a glass plate by means of an applicator so as to have a dry thickness of 50 μm, and air-dried at 23°C and 50%RH for 1 day, to thereby prepare a test piece. The thus-prepared test piece was immersed in distilled water for 1 day, and occurrence of falling and peeling of coating film were evaluated on the basis of the following ratings.

⊚: No change was observed in the coating film (good)
O: Slight peeling or falling was observed in the coating film (fair)
Δ: Peeling or falling was observed in about a half portion of the coating film (slightly bad)
X: Peeling or falling was observed in a considerable portion of the coating film (bad)

<Oil resistance>

A test piece prepared through the same method as employed in the aforementioned water resistance test was immersed in oleic acid for 1 day, and occurrence of falling and peeling coating film were evaluated on the basis of the same ratings as employed in the aforementioned water resistance test.

<Cosmetic effect consistency (cosmetic persistency)>

Ten panelists joined to a test of usability for evaluating cosmetic persistency (i.e., cosmetic effect consistency). The ratings of evaluation are as follows.

⊚: All 10 panelists evaluated as good.
O: ≥8 of 10 Panelists evaluated as good.
Δ: ≥5 of 10 Panelists evaluated as good.
X: ≤4 of 10 Panelists evaluated as good.

<Stability over time>
A cosmetic composition (40 g) was placed in a glass container (50 mL) and closely sealed in the container with a cap. The container was maintained in a hot air drier at 40°C for 1 month. Thereafter, the state of separation was observed to evaluate stability over time, which was evaluated on the basis of the following ratings.

(Ratings of evaluation)

⊚: no separation (very good)
O: slight separation, but disappeared in mixing (good)
Δ: separation, and not disappeared in mixing (slightly bad)
X: considerable separation (bad)

[Table 5]

| | Examples | | | | | | | | | | | | | | | | | Comparative Examples | | | | | |
|---|---|---|---|---|---|---|---|---|---|---|---|---|---|---|---|---|---|---|---|---|---|---|---|
| | 34 | 35 | 36 | 37 | 38 | 39 | 40 | 41 | 42 | 43 | 44 | 45 | 46 | 47 | 48 | 49 | 50 | 7 | 8 | 9 | 10 | 11 | 12 |
| Aq. emulsion obtained in Ex. 1 | 45 | | | | | | | | | | | | | | | | | | | | | | |
| Aq. emulsion obtained in Ex. 3 | | 45 | | | | | | | | | | | | | | | | | | | | | |
| Aq. emulsion obtained in Ex. 5 | | | 45 | | | | | | | | | | | | | | | | | | | | |
| Aq. emulsion obtained in Ex. 7 | | | | 45 | | | | | | | | | | | | | | | | | | | |
| Aq. emulsion obtained in Ex. 9 | | | | | 45 | | | | | | | | | | | | | | | | | | |
| Aq. emulsion obtained in Ex. 11 | | | | | | 45 | | | | | | | | | | | | | | | | | |
| Aq. emulsion obtained in Ex. 13 | | | | | | | 45 | | | | | | | | | | | | | | | | |
| Aq. emulsion obtained in Ex. 15 | | | | | | | | 45 | | | | | | | | | | | | | | | |
| Aq. emulsion obtained in Ex. 17 | | | | | | | | | 45 | | | | | | | | | | | | | | |
| Aq. emulsion obtained in Ex. 19 | | | | | | | | | | 45 | | | | | | | | | | | | | |
| Aq. emulsion obtained in Ex. 21 | | | | | | | | | | | 45 | | | | | | | | | | | | |
| Aq. emulsion obtained in Ex. 23 | | | | | | | | | | | | 45 | | | | | | | | | | | |
| Aq. emulsion obtained in Ex. 25 | | | | | | | | | | | | | 45 | | | | | | | | | | |
| Aq. emulsion obtained in Ex. 27 | | | | | | | | | | | | | | 45 | | | | | | | | | |
| Aq. emulsion obtained in Ex. 29 | | | | | | | | | | | | | | | 45 | | | | | | | | |
| Aq. emulsion obtained in Ex. 31 | | | | | | | | | | | | | | | | 45 | | | | | | | |
| Aq. emulsion obtained in Ex. 33 | | | | | | | | | | | | | | | | | 45 | | | | | | |
| Aq. emulsion obtained in Comp. Ex. 1 | | | | | | | | | | | | | | | | | | 45 | | | | | |
| Aq. emulsion obtained in Comp. Ex. 2 | | | | | | | | | | | | | | | | | | | 45 | | | | |
| Aq. emulsion obtained in Comp. Ex. 3 | | | | | | | | | | | | | | | | | | | | 45 | | | |
| Aq. emulsion obtained in Comp. Ex. 4 | | | | | | | | | | | | | | | | | | | | | 45 | | |
| Aq. emulsion obtained in Comp. Ex. 5 | | | | | | | | | | | | | | | | | | | | | | 45 | |
| Aq. emulsion obtained in Comp. Ex. 6 | | | | | | | | | | | | | | | | | | | | | | | 45 |
| Black iron oxide | 15 | 15 | 15 | 15 | 15 | 15 | 15 | 15 | 15 | 15 | 15 | 15 | 15 | 15 | 15 | 15 | 15 | 15 | 15 | 15 | 16 | 15 | 15 |
| Talc | 10 | 10 | 10 | 10 | 10 | 10 | 10 | 10 | 10 | 10 | 10 | 10 | 10 | 10 | 10 | 10 | 10 | 10 | 10 | 10 | 11 | 10 | 10 |
| Methylhydroxypropylcellulose | 2 | 2 | 2 | 2 | 2 | 2 | 2 | 2 | 2 | 2 | 2 | 2 | 2 | 2 | 2 | 2 | 2 | 2 | 2 | 2 | 3 | 2 | 2 |
| Polyoxyethylene sorbitan monooleate | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 1.5 | 2.5 | 1.5 | 1.5 |
| Glycerin | 5 | 5 | 5 | 5 | 5 | 5 | 5 | 5 | 5 | 5 | 5 | 5 | 5 | 5 | 5 | 5 | 5 | 5 | 5 | 5 | 6 | 5 | 5 |
| Ion-exchanged water | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 22.3 | 23.3 | 22.3 | 22.3 |
| Perfume | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 1.1 | 0.1 | 0.1 |
| Antiseptic | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 1.1 | 0.1 | 0.1 |
| Evaluation — Water resistance | ◎ | ◎ | ◎ | ◎ | ◎ | ◎ | ◎ | ◎ | ◎ | ○ | ○ | ○ | ○ | ◎ | ◎ | ◎ | ◎ | ○ | × | × | △ | × | × |
| Evaluation — Oil resistance | ◎ | ◎ | ○ | ◎ | ◎ | ◎ | ◎ | ◎ | ◎ | ◎ | ◎ | ○ | ○ | ◎ | ○ | ○ | ◎ | × | ○ | ○ | × | × | × |
| Evaluation — Cosmetic persistency | ◎ | ◎ | ○ | ◎ | ◎ | ◎ | ◎ | ◎ | ◎ | ◎ | ◎ | ○ | ○ | ◎ | ○ | ○ | ◎ | △ | △ | △ | × | × | × |
| Evaluation — Stability over time | ◎ | ◎ | ◎ | ◎ | ◎ | ◎ | ◎ | ◎ | ◎ | ◎ | ◎ | ◎ | ◎ | ◎ | ◎ | ○ | ◎ | ◎ | △ | ◎ | ◎ | ◎ | ◎ |

As shown in Table 5, the cosmetic compositions of Examples 34 to 50 were found to exhibit excellent water resistance, oil resistance, and cosmetic persistency, as compared with Comparative Example 7 (the composition of monomer (A) falling outside the scope of the present disclosure); Comparative Example 8 (the composition of monomer (B) falling outside the scope of the present disclosure); Comparative Example 9 (a volume average particle size less than 70 nm); Comparative Examples 10 and 12 (a volume average particle size in excess of 150 nm); and Comparative Example 11 (a particle size distribution factor greater than 1.3 and a volume average particle size in excess of 150 nm). In addition, the cosmetic compositions of Examples 34 to 50 were also found to exhibit excellent stability over time.

[Example 51]

Production and evaluation of manicure

A solution prepared by mixing ethylene glycol monoethyl ether (10 parts), acetyl tributyl citrate (3 parts), and ion-exchanged water (7 parts) was gradually added to the aqueous polymer emulsion (80 parts) obtained in Example 2, and stirring was continued to achieve a uniform state, to thereby yield a manicure. The thus-obtained manicure was evaluated in terms of water resistance, oil resistance, cosmetic persistency, and stability over time, through the same evaluation method as employed in the case of mascaras.

[Examples 52 to 66 and Comparative Examples 13 to 18]

Production and evaluation of manicures

The procedure of Example 51 was repeated, except that the emulsion was changed to a corresponding aqueous polymer emulsion shown in Table 6, to thereby yield manicures of Examples 52 to 66 and Comparative Examples 13 to 18. Also, each of the thus-obtained manicures was evaluated in terms of water resistance, oil resistance, cosmetic persistency, and stability over time, through the same evaluation method as employed in the case of mascaras.

[Table 6]

| | Examples | | | | | | | | | | | | | | | | Comparative Examples | | | | | |
|---|---|---|---|---|---|---|---|---|---|---|---|---|---|---|---|---|---|---|---|---|---|---|---|
| | 51 | 52 | 53 | 54 | 55 | 56 | 57 | 58 | 59 | 60 | 61 | 62 | 63 | 64 | 65 | 66 | 13 | 14 | 15 | 16 | 17 | 18 |
| Aq. emulsion obtained in Ex. 2 | 80 | | | | | | | | | | | | | | | | | | | | | |
| Aq. emulsion obtained in Ex. 4 | | 80 | | | | | | | | | | | | | | | | | | | | |
| Aq. emulsion obtained in Ex. 6 | | | 80 | | | | | | | | | | | | | | | | | | | |
| Aq. emulsion obtained in Ex. 8 | | | | 80 | | | | | | | | | | | | | | | | | | |
| Aq. emulsion obtained in Ex. 10 | | | | | 80 | | | | | | | | | | | | | | | | | |
| Aq. emulsion obtained in Ex. 12 | | | | | | 80 | | | | | | | | | | | | | | | | |
| Aq. emulsion obtained in Ex. 14 | | | | | | | 80 | | | | | | | | | | | | | | | |
| Aq. emulsion obtained in Ex. 16 | | | | | | | | 80 | | | | | | | | | | | | | | |
| Aq. emulsion obtained in Ex. 18 | | | | | | | | | 80 | | | | | | | | | | | | | |
| Aq. emulsion obtained in Ex. 20 | | | | | | | | | | 80 | | | | | | | | | | | | |
| Aq. emulsion obtained in Ex. 22 | | | | | | | | | | | 80 | | | | | | | | | | | |
| Aq. emulsion obtained in Ex. 24 | | | | | | | | | | | | 80 | | | | | | | | | | |
| Aq. emulsion obtained in Ex. 26 | | | | | | | | | | | | | 80 | | | | | | | | | |
| Aq. emulsion obtained in Ex. 28 | | | | | | | | | | | | | | 80 | | | | | | | | |
| Aq. emulsion obtained in Ex. 30 | | | | | | | | | | | | | | | 80 | | | | | | | |
| Aq. emulsion obtained in Ex. 32 | | | | | | | | | | | | | | | | 80 | | | | | | |
| Aq. emulsion obtained in Comp. Ex. 1 | | | | | | | | | | | | | | | | | 80 | | | | | |
| Aq. emulsion obtained in Comp. Ex. 2 | | | | | | | | | | | | | | | | | | 80 | | | | |
| Aq. emulsion obtained in Comp. Ex. 3 | | | | | | | | | | | | | | | | | | | 80 | | | |
| Aq. emulsion obtained in Comp. Ex. 4 | | | | | | | | | | | | | | | | | | | | 80 | | |
| Aq. emulsion obtained in Comp. Ex. 5 | | | | | | | | | | | | | | | | | | | | | 80 | |
| Aq. emulsion obtained in Comp. Ex. 6 | | | | | | | | | | | | | | | | | | | | | | 80 |
| Ethylene glycol monoethyl ether | 10 | 10 | 10 | 10 | 10 | 10 | 10 | 10 | 10 | 10 | 10 | 10 | 10 | 10 | 10 | 10 | 10 | 10 | 10 | 10 | 10 | 10 |
| Acetyl tributyl citrate | 3 | 3 | 3 | 3 | 3 | 3 | 3 | 3 | 3 | 3 | 3 | 3 | 3 | 3 | 3 | 3 | 3 | 3 | 3 | 3 | 3 | 3 |
| Ion-exchanged water | 7 | 7 | 7 | 7 | 7 | 7 | 7 | 7 | 7 | 7 | 7 | 7 | 7 | 7 | 7 | 7 | 7 | 7 | 7 | 7 | 7 | 7 |
| Evaluation — Water resistance | ◎ | ◎ | ◎ | ○ | ◎ | ○ | ◎ | ◎ | ◎ | ○ | ○ | ○ | ◎ | ◎ | ◎ | ◎ | △ | × | × | △ | × | × |
| Evaluation — Oil resistance | ◎ | ◎ | ○ | ◎ | ◎ | ○ | ◎ | ◎ | ◎ | ○ | ○ | ◎ | ◎ | ◎ | ○ | ◎ | × | △ | ○ | × | × | × |
| Evaluation — Cosmetic persistency | ◎ | ◎ | ◎ | ◎ | ◎ | ○ | ◎ | ◎ | ◎ | ◎ | ◎ | ○ | ◎ | ◎ | ○ | ○ | △ | △ | △ | × | × | × |
| Evaluation — Stability over time | ◎ | ◎ | ○ | ○ | ◎ | ◎ | ◎ | ◎ | ◎ | ◎ | ◎ | ◎ | ◎ | ◎ | ◎ | ◎ | ◎ | △ | ◎ | ◎ | ◎ | ◎ |

As shown in Table 6, the cosmetic compositions of Examples 51 to 66 were found to exhibit more excellent water resistance, oil resistance, and cosmetic persistency, as compared with Comparative Example 13 (the composition of monomer (A) falling outside the scope of the present disclosure); Comparative Example 14 (the composition of monomer (B) falling outside the scope of the present disclosure); Comparative Example 15 (a volume average particle size less than 70 nm); Comparative Examples 16 and 18 (a volume average particle size in excess of 150 nm); and Comparative Example 17 (a particle size distribution factor greater than 1.3 and a volume average particle size in excess of 150 nm). In addition, the cosmetic compositions of Examples 51 to 66 were also found to exhibit excellent results in terms of stability over time.

[Example 67]

Production and evaluation of eyeliner

Polyoxyethylene (20) sorbitan monooleate (1 part), carboxymethylcellulose (0.15 parts), and iron oxide (14 parts) were added to ion-exchanged water (33.9 parts), and the mixture was uniformly mixed through stirring. Subsequently, glycerin (5 parts) and acetyl tributyl citrate (1 part) were added thereto, and the aqueous polymer emulsion obtained in Example 1 (45 parts) was gradually added. The formulation was stirred to achieve a uniform state, to thereby yield an eyeliner. The thus-obtained eyeliner was evaluated in terms of water resistance, oil resistance, cosmetic persistency, and stability over time, through the same evaluation method as employed in the case of mascaras.

[Examples 67 to 83 and Comparative Examples 19 to 24]

Production and evaluation of eyeliners

The procedure of Example 68 was repeated, except that the formulation was changed to a corresponding one shown in Table 7, to thereby yield eyeliners of Examples 68 to 83 and Comparative Examples 19 to 24. Also, each of the thus-obtained eyeliners was evaluated in terms of water resistance, oil resistance, cosmetic persistency, and stability over time, through the same evaluation method as employed in the case of mascaras.

[Table 7]

|  | Examples | | | | | | | | | | | | | | | | | Comparative Examples | | | | | |
|---|---|---|---|---|---|---|---|---|---|---|---|---|---|---|---|---|---|---|---|---|---|---|---|
|  | 67 | 68 | 69 | 70 | 71 | 72 | 73 | 74 | 75 | 76 | 77 | 78 | 79 | 80 | 81 | 82 | 83 | 19 | 20 | 21 | 22 | 23 | 24 |
| Aq. emulsion obtained in Ex. 1 | 45 | | | | | | | | | | | | | | | | | | | | | | |
| Aq. emulsion obtained in Ex. 3 | | 45 | | | | | | | | | | | | | | | | | | | | | |
| Aq. emulsion obtained in Ex. 5 | | | 45 | | | | | | | | | | | | | | | | | | | | |
| Aq. emulsion obtained in Ex. 7 | | | | 45 | | | | | | | | | | | | | | | | | | | |
| Aq. emulsion obtained in Ex. 9 | | | | | 45 | | | | | | | | | | | | | | | | | | |
| Aq. emulsion obtained in Ex. 11 | | | | | | 45 | | | | | | | | | | | | | | | | | |
| Aq. emulsion obtained in Ex. 13 | | | | | | | 45 | | | | | | | | | | | | | | | | |
| Aq. emulsion obtained in Ex. 15 | | | | | | | | 45 | | | | | | | | | | | | | | | |
| Aq. emulsion obtained in Ex. 17 | | | | | | | | | 45 | | | | | | | | | | | | | | |
| Aq. emulsion obtained in Ex. 19 | | | | | | | | | | 45 | | | | | | | | | | | | | |
| Aq. emulsion obtained in Ex. 21 | | | | | | | | | | | 45 | | | | | | | | | | | | |
| Aq. emulsion obtained in Ex. 23 | | | | | | | | | | | | 45 | | | | | | | | | | | |
| Aq. emulsion obtained in Ex. 25 | | | | | | | | | | | | | 45 | | | | | | | | | | |
| Aq. emulsion obtained in Ex. 27 | | | | | | | | | | | | | | 45 | | | | | | | | | |
| Aq. emulsion obtained in Ex. 29 | | | | | | | | | | | | | | | 45 | | | | | | | | |
| Aq. emulsion obtained in Ex. 31 | | | | | | | | | | | | | | | | 45 | | | | | | | |
| Aq. emulsion obtained in Ex. 33 | | | | | | | | | | | | | | | | | 45 | | | | | | |
| Aq. emulsion obtained in Comp. Ex. 1 | | | | | | | | | | | | | | | | | | 45 | | | | | |
| Aq. emulsion obtained in Comp. Ex. 2 | | | | | | | | | | | | | | | | | | | 45 | | | | |
| Aq. emulsion obtained in Comp. Ex. 3 | | | | | | | | | | | | | | | | | | | | 45 | | | |
| Aq. emulsion obtained in Comp. Ex. 4 | | | | | | | | | | | | | | | | | | | | | 45 | | |
| Aq. emulsion obtained in Comp. Ex. 5 | | | | | | | | | | | | | | | | | | | | | | 45 | |
| Aq. emulsion obtained in Comp. Ex. 6 | | | | | | | | | | | | | | | | | | | | | | | 45 |
| Iron oxide | 14 | 14 | 14 | 14 | 14 | 14 | 14 | 14 | 14 | 14 | 14 | 14 | 14 | 14 | 14 | 14 | 14 | 14 | 14 | 14 | 14 | 14 | 14 |
| Glycerin | 5 | 5 | 5 | 5 | 5 | 5 | 5 | 5 | 5 | 5 | 5 | 5 | 5 | 5 | 5 | 5 | 5 | 5 | 5 | 5 | 5 | 5 | 5 |
| Polyoxyethylene(20) sorbitan monooleate ester | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Carboxymethylcellulose | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 | 0.15 |
| Acetyl tributyl citrate | 1 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Ion-exchanged water | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 | 33.9 |
| Evaluation — Water resistance | ◎ | ◎ | ◎ | ◎ | ◎ | ◎ | ◎ | ◎ | ○ | ◎ | ○ | ○ | ○ | ○ | ○ | ◎ | ◎ | △ | × | × | △ | × | × |
| Evaluation — Oil resistance | ◎ | ◎ | ○ | ◎ | ◎ | ◎ | ◎ | ◎ | ◎ | ◎ | ◎ | ○ | ○ | ◎ | ◎ | ○ | ◎ | △ | ○ | △ | × | × | × |
| Evaluation — Cosmetic persistency | ◎ | ◎ | ○ | ◎ | ◎ | ◎ | ◎ | ◎ | ◎ | ◎ | ◎ | ○ | ○ | ◎ | ○ | ○ | ◎ | △ | △ | △ | × | × | × |
| Evaluation — Stability over time | ◎ | ◎ | ◎ | ◎ | ◎ | ◎ | ◎ | ◎ | ◎ | ◎ | ◎ | ◎ | ◎ | ◎ | ◎ | ○ | ◎ | ◎ | △ | ◎ | ◎ | ◎ | ◎ |

As shown in Table 7, the cosmetic compositions of Examples 67 to 83 were found to exhibit more excellent water resistance, oil resistance, and cosmetic persistency, as compared with Comparative Example 19 (the composition of monomer (A) falling outside the scope of the present disclosure); Comparative Example 20 (the composition of monomer (B) falling outside the scope of the present disclosure); Comparative Example 21 (a volume average particle size less than 70 nm); Comparative Examples 22 and 24 (a volume average particle size in excess of 150 nm); and Comparative Example 23 (a particle size distribution factor greater than 1.3 and a volume average particle size in excess of 150 nm). In addition, the cosmetic compositions of Examples 67 to 83 were also found to exhibit excellent results in terms of stability over time.

[Example 84]

Production and evaluation of foundation

The aqueous polymer emulsion obtained in Example 2 (5 parts), bentonite (0.5 parts), polyoxyethylene sorbitan monostearate (1 part), propylene glycol (10 parts), an antiseptic (0.1 parts), and ion-exchanged water (52.4 parts) were mixed, and the ingredients were dispersed to a uniform state at 70°C by means of a homo mixer. The pH of the mixture was adjusted to 7.0 with triethanolamine. Subsequently, talc (3 parts), titanium dioxide (5 parts), red iron oxide (0.5 parts), and iron oxide (1 part) were sufficiently mixed, and the mixture was added to the above-prepared dispersion. Stearic acid (2.5 parts), isohexadecyl alcohol (7 parts), glycerin monostearate (2 parts), liquid lanolin (2 parts), and liquid paraffin (8 parts) were mixed, and the ingredients were dissolved at 80°C, to thereby form an oil phase. The oil phase was added to the above-prepared aqueous phase, and the mixture was processed by means of a homo mixer at 70°C for emulsification. Thereafter, the emulsion was cooled to ambient temperature, to thereby yield a foundation. The thus-obtained foundation was evaluated in terms of water resistance, oil resistance, cosmetic persistency, and stability over time, through the same evaluation method as employed in the case of mascaras.

[Examples 85 to 99 and Comparative Examples 25 to 30]

Production and evaluation of foundations

The procedure of Example 84 was repeated, except that the formulation was changed to a corresponding one shown in Table 8, to thereby yield foundations of Examples 85 to 99 and Comparative Examples 25 to 30. Also, each of the thus-obtained foundations was evaluated in terms of water resistance, oil resistance, cosmetic persistency, and stability over time, through the same evaluation method as employed in the case of mascaras.

[Table 8]

| | Examples | | | | | | | | | | | | | | | | Comparative Examples | | | | | |
|---|---|---|---|---|---|---|---|---|---|---|---|---|---|---|---|---|---|---|---|---|---|---|
| | 84 | 85 | 86 | 87 | 88 | 89 | 90 | 91 | 92 | 93 | 94 | 95 | 96 | 97 | 98 | 99 | 25 | 26 | 27 | 28 | 29 | 30 |
| Aq; emulsion obtained in Ex. 2 | 5 | | | | | | | | | | | | | | | | | | | | | |
| Aq; emulsion obtained in Ex. 4 | | 5 | | | | | | | | | | | | | | | | | | | | |
| Aq; emulsion obtained in Ex. 6 | | | 5 | | | | | | | | | | | | | | | | | | | |
| Aq; emulsion obtained in Ex. 8 | | | | 5 | | | | | | | | | | | | | | | | | | |
| Aq; emulsion obtained in Ex. 10 | | | | | 5 | | | | | | | | | | | | | | | | | |
| Aq; emulsion obtained in Ex. 12 | | | | | | 5 | | | | | | | | | | | | | | | | |
| Aq; emulsion obtained in Ex. 14 | | | | | | | 5 | | | | | | | | | | | | | | | |
| Aq; emulsion obtained in Ex. 16 | | | | | | | | 5 | | | | | | | | | | | | | | |
| Aq; emulsion obtained in Ex. 18 | | | | | | | | | 5 | | | | | | | | | | | | | |
| Aq; emulsion obtained in Ex. 20 | | | | | | | | | | 5 | | | | | | | | | | | | |
| Aq; emulsion obtained in Ex. 22 | | | | | | | | | | | 5 | | | | | | | | | | | |
| Aq; emulsion obtained in Ex. 24 | | | | | | | | | | | | 5 | | | | | | | | | | |
| Aq; emulsion obtained in Ex. 26 | | | | | | | | | | | | | 5 | | | | | | | | | |
| Aq; emulsion obtained in Ex. 28 | | | | | | | | | | | | | | 5 | | | | | | | | |
| Aq; emulsion obtained in Ex. 30 | | | | | | | | | | | | | | | 5 | | | | | | | |
| Aq; emulsion obtained in Ex. 32 | | | | | | | | | | | | | | | | 5 | | | | | | |
| Aq; emulsion obtained in Comp. Ex. 1 | | | | | | | | | | | | | | | | | 5 | | | | | |
| Aq; emulsion obtained in Comp. Ex. 2 | | | | | | | | | | | | | | | | | | 5 | | | | |
| Aq; emulsion obtained in Comp. Ex. 3 | | | | | | | | | | | | | | | | | | | 5 | | | |
| Aq; emulsion obtained in Comp. Ex. 4 | | | | | | | | | | | | | | | | | | | | 5 | | |
| Aq; emulsion obtained in Comp. Ex. 5 | | | | | | | | | | | | | | | | | | | | | 5 | |
| Aq; emulsion obtained in Comp. Ex. 6 | | | | | | | | | | | | | | | | | | | | | | 5 |
| Bentonite | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Polyoxyethylene sorbitan monostearate | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Propylene glycol | 10 | 10 | 10 | 10 | 10 | 10 | 10 | 10 | 10 | 10 | 10 | 10 | 10 | 10 | 10 | 10 | 10 | 10 | 10 | 10 | 10 | 10 |
| Triethanolamine (to pH 7) | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount | appropriate amount |
| Antiseptic | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| Ion-exchanged water | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 | 52.4 |
| Talc | 3 | 3 | 3 | 3 | 3 | 3 | 3 | 3 | 3 | 3 | 3 | 3 | 3 | 3 | 3 | 3 | 3 | 3 | 3 | 3 | 3 | 3 |
| Titanium dioxide | 5 | 5 | 5 | 5 | 5 | 5 | 5 | 5 | 5 | 5 | 5 | 5 | 5 | 5 | 5 | 5 | 5 | 5 | 5 | 5 | 5 | 5 |
| Red iron oxide | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Iron oxide | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| Stearic acid | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 | 2.5 |
| Isohexadecyl alcohol | 7 | 7 | 7 | 7 | 7 | 7 | 7 | 7 | 7 | 7 | 7 | 7 | 7 | 7 | 7 | 7 | 7 | 7 | 7 | 7 | 7 | 7 |
| Glycerin monostearate | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| Liq. Lanoline | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| Liq. Paraffin | 8 | 8 | 8 | 8 | 8 | 8 | 8 | 8 | 8 | 8 | 8 | 8 | 8 | 8 | 8 | 8 | 8 | 8 | 8 | 8 | 8 | 8 |
| Evaluation — Water resistance | ◎ | ◎ | ○ | ◎ | ○ | ○ | ◎ | ◎ | ◎ | ○ | ○ | ○ | ○ | ◎ | ○ | ◎ | ○ | × | × | △ | × | × |
| Evaluation — Oil resistance | ◎ | ○ | ◎ | ◎ | ◎ | ○ | ◎ | ○ | ○ | ○ | ○ | ◎ | ◎ | ◎ | ◎ | ◎ | × | △ | × | × | × | × |
| Evaluation — Cosmetic persistency | ◎ | ○ | ◎ | ◎ | ◎ | ○ | ○ | ◎ | ○ | ◎ | ◎ | ◎ | ○ | ◎ | ◎ | ○ | △ | △ | △ | × | × | × |
| Evaluation — Stability over time | ◎ | ◎ | ◎ | ◎ | ◎ | ○ | ◎ | ◎ | ◎ | ◎ | ◎ | ◎ | ◎ | ◎ | ○ | ◎ | ◎ | △ | ◎ | ◎ | ◎ | ◎ |

As shown in Table 8, the cosmetic compositions of Examples 84 to 99 were found to exhibit more excellent water resistance, oil resistance, and cosmetic persistency, as compared with Comparative Example 25 (the composition of monomer (A) falling outside the scope of the present disclosure); Comparative Example 26 (the composition of monomer (B) falling outside the scope of the present disclosure); Comparative Example 27 (a volume average particle size less than 70 nm); Comparative Examples 28 and 30 (a volume average particle size in excess of 150 nm); and Comparative Example 29 (a particle size distribution factor greater than 1.3 and a volume average particle size in excess of 150 nm). In addition, the cosmetic compositions of Examples 84 to 99 were also found to exhibit excellent results in terms of stability over time.

The present invention should not be limited to the aforementioned embodiments and encompasses various modifications and modification within a scope of equivalent, so long as they do not deviate from the gist of the present invention. Thus, those skilled in the art should understand that, in reference to the aforementioned teachings, various combinations and modes, and further, those including only one element, one or more elements, or one or less element fall within the scope or concept of the present invention.

**Claims**

1. An aqueous polymer emulsion for cosmetics, the emulsion comprising resin particles, wherein

   the resin particles comprise a structural unit derived from a monomer (A) in an amount of 65 to 99.9 mass%, and a structural unit derived from a monomer (B) in an amount of 0.1 to 10 mass%, with respect to the total amount of the structural units derived from the monomers forming the resin particles as 100 mass%, wherein
   the monomer (A) is an alkyl (meth)acrylate having an C3 to C8 alkyl group; and
   the monomer (B) is at least one monomer selected from the group consisting of an unsaturated carboxylic acid and an unsaturated carboxylic anhydride; and
   the resin particles have a volume average particle size of 70 to 150 nm and a ratio of the volume average particle size to a cumulative average particle size (volume average particle size/cumulative average particle size) of 1.00 to 1.30.

2. The aqueous polymer emulsion for cosmetics according to claim 1, wherein the polymer forming the resin particles has a glass transition temperature of -20°C to 20°C.

3. The aqueous polymer emulsion for cosmetics according to claim 1, wherein the monomer (B) is methacrylic acid.

4. The aqueous polymer emulsion for cosmetics according to claim 1, which emulsion has a volatile organic compound level of 100 ppm or lower.

5. A method for producing the aqueous polymer emulsion for cosmetics according to claim 1, the method comprising

   a first polymerization step of adding a monomer into a reaction container and conducting emulsion polymerization, under such conditions that a C1 to C4 lower alcohol is present at a level of 10,000 ppm or lower in the reaction container and an anionic surfactant is present at an amount of 0.15 to 0.80 parts by mass, with respect to the total amount of monomers used in production of the resin particles as 100 parts by mass, to thereby produce seed particles; and
   a second polymerization step of adding a monomer into a reaction container and conducting emulsion polymerization in the presence of the seed particles.

6. The method according to claim 5 for producing the aqueous polymer emulsion for cosmetics, wherein,

   in the first polymerization step, the lower alcohol is present in the reaction container, and
   the lower alcohol is ethanol.

7. A method for producing an odor-reduced aqueous polymer emulsion for cosmetics, the method comprising a step comprising placing the aqueous polymer emulsion for cosmetics produced through a production method according to claim 5 or 6 in a processing container that can provide reduced pressure; and supplying pressurized steam to the processing container, to thereby remove a volatile organic compound present in the aqueous polymer emulsion for cosmetics, while the temperature of the emulsion in the processing container is controlled to 50°C to 85°C; the pressure in the processing container is controlled to 12 kPa to 57 kPa; and the internal state of the processing container is maintained under a water boiling condition.

8. A cosmetic comprising the aqueous polymer emulsion for cosmetics according to any one of claims 1 to 4.

## INTERNATIONAL SEARCH REPORT

| | International application No. |
|---|---|
| | **PCT/JP2023/038015** |

| A. CLASSIFICATION OF SUBJECT MATTER |
|---|
| *A61K 8/81*(2006.01)i; *A61K 8/06*(2006.01)i; *A61Q 1/00*(2006.01)i; *A61Q 5/00*(2006.01)i; *A61Q 17/04*(2006.01)i; *A61Q 19/00*(2006.01)i <br> FI:    A61K8/81; A61K8/06; A61Q1/00; A61Q19/00; A61Q5/00; A61Q17/04 |
| According to International Patent Classification (IPC) or to both national classification and IPC |

| B. FIELDS SEARCHED |
|---|
| Minimum documentation searched (classification system followed by classification symbols) <br>     A61K8/81; A61K8/06; A61Q1/00; A61Q5/00; A61Q17/04; A61Q19/00 |
| Documentation searched other than minimum documentation to the extent that such documents are included in the fields searched <br>     Published examined utility model applications of Japan 1922-1996 <br>     Published unexamined utility model applications of Japan 1971-2023 <br>     Registered utility model specifications of Japan 1996-2023 <br>     Published registered utility model applications of Japan 1994-2023 |
| Electronic data base consulted during the international search (name of data base and, where practicable, search terms used) |

| C. DOCUMENTS CONSIDERED TO BE RELEVANT |
|---|

| Category* | Citation of document, with indication, where appropriate, of the relevant passages | Relevant to claim No. |
|---|---|---|
| X | WO 2021/095604 A1 (TOAGOSEI CO., LTD.) 20 May 2021 (2021-05-20) <br>     claims, paragraphs [0010], [0012], [0071], [0090], [0091] | 1-8 |
| X | WO 2021/241091 A1 (NIKKO CHEMICALS) 02 December 2021 (2021-12-02) <br>     claims, paragraphs [0006], [0007], [0021], [0022], [0062], [0063], [0086], [0087] | 1-8 |
| A | JP 1-203313 A (SHISEIDO CO., LTD.) 16 August 1989 (1989-08-16) <br>     entire text | 1-8 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 December 2023** | **26 December 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT
### Information on patent family members

International application No.

**PCT/JP2023/038015**

| Patent document cited in search report | Publication date (day/month/year) | Patent family member(s) | Publication date (day/month/year) |
|---|---|---|---|
| WO 2021/095604 A1 | 20 May 2021 | (Family: none) | |
| WO 2021/241091 A1 | 02 December 2021 | (Family: none) | |
| JP 1-203313 A | 16 August 1989 | (Family: none) | |

Form PCT/ISA/210 (patent family annex) (January 2015)

**EP 4 609 849 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022172405 A
- JP 54049338 A
- JP 2002327019 A
- JP 2000355532 A